# EUROPÄISCHE PATENTSCHRIFT

(11) **EP 2 683 704 B1**
(45) Veröffentlichungstag und Bekanntmachung des Hinweises auf die Patenterteilung: **17.12.2014**
(21) Anmeldenummer: 12707339.3
(22) Anmeldetag: 07.03.2012
(51) Int. Cl.: C07D 291/08, A61K 31/54, A61P 3/10

(54) **VERZWEIGTE OXATHIAZINDERIVATE, VERFAHREN ZU DEREN HERSTELLUNG, IHRE VERWENDUNG ALS MEDIKAMENT SOWIE SIE ENTHALTENDES ARZNEIMITTEL UND DEREN VERWENDUNG**
BRANCHED OXATHIAZINE DERIVATIVES, METHOD FOR THE PRODUCTION THEREOF, USE THEREOF AS MEDICINE AND DRUG CONTAINING SAID DERIVATIVES AND USE THEREOF
DÉRIVÉS OXATHIAZINE RAMIFIÉS, PROCÉDÉ POUR LEUR PRÉPARATION, UTILISATION EN TANT QUE MÉDICAMENT, AGENTS PHARMACEUTIQUES CONTENANT CES DÉRIVÉS ET LEUR UTILISATION

(30) Priorität: 08.03.2011 EP 11305241
(43) Veröffentlichungstag der Anmeldung: 15.01.2014
(73) Patentinhaber: SANOFI, 75008 Paris (FR)
(72) Erfinder: BOEHME, Thomas, 65926 Frankfurt am Main (DE); ENGEL, Christian, 65926 Frankfurt am Main (DE); GUESSREGEN, Stefan, 65926 Frankfurt am Main (DE); HAACK, Torsten, 65926 Frankfurt am Main (DE); RITTER, Kurt, 65926 Frankfurt am Main (DE); TSCHANK, Georg, 65926 Frankfurt am Main (DE)
(74) Vertreter: Essler, Frank
(86) Internationale Anmeldenummer: PCT/EP2012/053936
(87) Internationale Veröffentlichungsnummer: WO 2012/120053

(56) Entgegenhaltungen:
- WO-A1-02/11722
- WO-A2-2008/073956
- SUZUE SEIGO ET AL: "Studies on Hypoglycemic Agents. IV. Synthesis of 1,4,3-Benzoxathiazine-4,4-dioxides", CHEMICAL AND PHARMACEUTICAL BULLETIN, PHARMACEUTICAL SOCIETY OF JAPAN, TOKYO, JP, Bd. 16, Nr. 5, 25. Mai 1968 (1968-05-25), Seiten 806-813, XP009109825, ISSN: 0009-2363
- IWAKAWA TSUNEO ET AL: "Cycloaddition in Synthesis of Sulfonamide Derivatives. IV. One-Pot Synthesis of 3-Dimethylamino-4,1,2-benzoxathiazine 1,1-Dioxides, 3-Methoxy-4-methyl-1,2,4-benzothiadiazine 1,1-Dioxide and 3-Dimethylamino-1,4,2-benzodithiazine 1,1-Dioxides", CHEMICAL AND PHARMACEUTICAL BULLETIN, PHARMACEUTICAL SOCIETY OF JAPAN, TOKYO, JP, Bd. 39, Nr. 8, 25. August 1991 (1991-08-25), Seiten 1939-1943, XP009109826, ISSN: 0009-2363

## Beschreibung

Die Erfindung betrifft substituierte Oxathiazinderivate und deren physiologisch verträgliche Salze.

In Suzue Seigo et al., Chem. Phar. Bull. 16(5), 806-813 (1986) sind Oxathiazinverbindungen zur Behandlung von Hypoglykämie beschrieben.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung von Diabetes, Hyperglykämie, Insulin Resistenz, Adipositas, Lipidstoffwechselstörungen oder anderen Erkrankungen geeignet sind.

Die Erfindung betrifft daher die Verbindung der Formel I 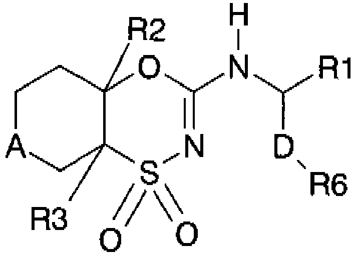 worin bedeuten
- A: CH₂, CF₂, O;
- D: (C₁-C₆)-Alkylen, (C₃-C₈)-Cycloalkylen, (C₁-C₆)-Alkylen-(C₃-C₈)-Cycloalkylen;
- R1: -(CH₂)ₙ-Aryl,
wobei der Arylrest ein bis 3-fach substituiert sein kann mit F, Cl, Br, I, OH, CF₃, CHF₂, CH₂F, NO₂, CN, OCF₃, QCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
-(CH₂)ₙ-Heteroaryl,
wobei der Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- n: 0, 1, 2;
- R2, R3: unabhängig voneinander H, (C₁-C₆)-Alkyl;
- R6: OH, O-(CO)-NH₂, SO₂NH₂;
sowie deren pharmazeutisch verträgliche Salze.

Bevorzugt sind Verbindungen der Formel I, worin bedeuten
- A: CH₂;
- D: (C₁-C₆)-Alkylen, (C₃-C₈)-Cycloalkylen;
- R1: -(CH₂)ₙ-Phenyl, -(CH₂)ₙ-Pyridinyl,
wobei der Phenylrest oder Pyridinylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, C₁-C₆)-Alkyl, NH₂, NH(C₁-C₆)-Alkyl, N((C₁-C₆)-Alkyls SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- n: 0, 1, 2;
- R2, R3: unabhängig voneinander H, (C₁-C₆)-Alkyl
- R6: OH, O-(CO)-NH₂, SO₂NH₂;
sowie deren pharmazeutisch verträgliche Salze.

Besonders bevorzugt sind Verbindungen der Formel I, worin bedeuten
- A: CH₂;
- D: (C₁-C₆)-Alkylen;
- R1: Phenyl, Pyridin
wobei der Phenylrest oder Pyridinylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2, R3: unabhängig voneinander H, (C₁-C₆)-Alkyl
- R6: OH;
sowie deren pharmazeutisch verträgliche Salze.

Ganz besonders bevorzugt sind Verbindungen der Formel I, worin bedeuten
- A: CH₂;
- D: (C₁-C₂)-Alkylen;
- R1: Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
- R2, R3: unabhängig voneinander H, (C₁-C₆)-Alkyl
- R6: OH;
sowie deren pharmazeutisch verträgliche Salze.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstofflkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.
Die Arylreste können ein oder mehrfach mit geeigneten Gruppen wie oben beschrieben substituiert sein.

Unter Heterozyklus bzw. heterozyklischer Rest werden Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heterocylus bzw. der heterocyclische Rest mit einem weiteren Ringsystem anelliert ist. Der Heterocylus bzw. der heterocyclische Rest kann gesättigt, teilweise gesättigt oder aromatisch sein.

Geeignete "Heterocyclen" bzw. "heterocyclische Reste" sind Acridinyl, Azepanyl, Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, 5,6-Dihydro-4H-cyclopentathiazol-2-yl, 4,5-Dihydro-thiazol-2-yl, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, 4,5,6,7-Tetrahydro-benzooxazol-2-yl, 4,5,6,7-Tetrahydro-benzothiazol-2-yl, 4,5,6,7-Tetrahydro-benzoimidazol-2-yl, 4,5,6,7-Tetrahydro-pyrazolo[1,5-a]pyridin-2-yl, Tetrahydrofuranyl, Tetrahydropyranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazinyl, Triazolyl, Tetrazolyl, Thiazolo[4,5-b]pyridinyl, Thieno[2,3-d]thiazol-2-yl, Tropanyl und Xanthenyl.

Die Heterozyklen bzw. heterozyklischen Reste können ein oder mehrfach mit geeigneten Gruppen wie oben beschrieben substituiert sein.,

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung. Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:
Alle Antidiabetika, die in der Roten Liste 2009, Kapitel 12 genannt sind; alle Abmagerungsmittel/Appetitzügler, die in der Roten Liste 2009, Kapitel 1 genannt sind; alle Diuretika, die in der Roten Liste 2009, Kapitel 36 genannt sind; alle Lipidsenker, die in der Roten Liste 2009, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Erfolgt die Gabe der Wirkstoffe durch getrennte Verabreichung der Wirkstoffe, so kann diese gleichzeitig oder nacheinander erfolgen. Die meisten der nachfolgend aufgeführten Wirkstoffe sind in USP Dictionary of USAN and International Drug Names, US Pharmacopeia, Rockville 2006, offenbart.

Antidiabetika umfassen Insulin und Insulinderivate, wie z.B. Lantus^{®} (siehe www.lantus.com) oder HMR 1964 oder Levemir® (insulin detemir), Humalog^{(R)} (Insulin Lispro), Humulin^{(R)}, VIAject™ oder solche, wie sie in WO2005005477 (Novo Nordisk) beschrieben sind, schnell wirkende Insuline (siehe US 6,221,633), inhalierbare Insuline, wie z. B. Exubera^{®}, Nasulin™, oder orale Insuline, wie z. B. IN-105 (Nobex) oder Oral-lyn ™ (Generex Biotechnology) oder Technosphere^{(R)} Insulin (MannKind) oder Cobalamin™ orales Insulin oder Insuline, wie sie in WO2007128815, WO2007128817 beschrieben sind oder Insuline, die transdermal verabreicht werden können;

GLP-1-Derivate und GLP-1 Agonisten wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), AVE-0010, BIM-51077 (R-1583, ITM-077), PC-DAC:Exendin-4 (ein Exendin-4 Analogon, welches kovalent an rekombinantes menschliches Albumin gebunden ist), CVX-73, CVX-98 und CVx-96 (GLP-1 Analoga, welche kovalent an einen monoklonalen Antikörper gebunden sind, der spezifische Bindungsstellen für das GLP-1 Peptid aufweist), CNTO-736 (ein GLP-1 Analogon, welches an eine Domäne gebunden ist, welche den Fc-Teil eines Antikörpers beinhaltet), PGC-GLP-1 (GLP-1 gebunden an einen Nanocarrier), Agonisten wie sie z.B. bei D. Chen et al., Proc. Natl. Acad. Sci. USA 104 (2007) 943 beschrieben sind, solche wie sie in WO2006124529, WO2007124461 beschrieben sind, Peptide wie z.B. Obinepitide (TM-30338), Amylinrezeptor Agonisten, wie sie z.B. in WO2007104789 beschrieben sind, Analoga des humanen GLP-1, wie sie in WO2007120899 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Antidiabetika umfassen auch Analoga und Derivate des Fibroblastenwachstumsfaktors 21 (FGF-21, fibroblast growth factor 21).

Die oral wirksamen hypoglykämischen Wirkstoffe umfassen vorzugsweise Sulfonylharnstoffe,
Biguanidine,
Meglitinide,
Oxadiazoli dindi one,
Thiazolidindione,
PPAR- und RXR-Modulatoren,
Glukosidase-Inhibitoren,
Hemmstoffe der Glykogenphosphorylase,
Glukagonrezeptor-Antagonisten,
Glukokinaseaktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT),
GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden,
Wirkstoffe, die auf den ATP-abhängigen Kaliumkanal der Betazellen wirken,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Modulatoren der natrium-abhängigen Glukosetransporter 1 oder 2 (SGLT1, SGLT2),
Hemmstoffe der 11-beta-Hydroxysteroid-Dehydrogenase-1 (11ß-HSD1),
Inhibitoren der Protein-Tyrosin-Phosphatase-1B (PTP-1B),
Nikotinsäurerezeptoragoni sten,
Inhibitoren der hormon-sensitiven bzw. endothelialen Lipasen,
Hemmstoffen der Acetyl-CoA Carboxylase (ACC1 und/oder ACC2) oder
Inhibitoren der GSK-3 beta.
Weiterhin sind umfasst den Fettstoffwechsel verändernde Verbindungen wie antihyperlipidämische Wirkstoffe und antilipidämische Wirkstoffe,
HMGCoA-Reduktase-Inhibitoren,
Farnesoid X Rezeptor (FXR) Antagonisten,
Fibrate,
Cholesterinresreptionsinhibitoren,
CETP-Inhibitoren,
Gallensäureresorptionsinhibitoren,
MTP-Inhibitoren,
Agonisten des Estrogenrezeptors gamma (ERR Agonisten),
Sigma-1 Rezeptorantagonisten,
Antagonisten des Somatostatin 5 Rezeptors (SST5 Rezeptor);
Verbindungen, die die Nahrungsmitteleinnahme verringern und
Verbindungen, die die Thermogenese erhöhen.

Bei einer Ausführungsform der Erfindung wird die Verbindung der Formel I in Kombination mit Insulin verabreicht.

Bei einer Ausführungsform wird die Verbindung der Formel I in Kombination mit einem Wirkstoff, der auf den ATP-abhängigen Kaliumkanal der Betazellen wirkt, z.B. Sulfonylharnstoffe, wie z.B. Tolbutamid, Glibenclamid, Glipizid, Gliclazide oder Glimepirid, verabreicht.

Bei einer Ausführungsform wird die Verbindung der Formel I in Kombination mit einer Tablette verabreicht, die sowohl Glimeprid enthält, welches schnell freigesetzt wird wie auch Metformin enthält, welches über einen längeren Zeitraum freigesetzt wird (wie z.B. in US2007264331 beschrieben).

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer weiteren Ausführungsform wird die Verbindung der Formel I in Kombination mit antidiabetischen Verbindungen, wie sie in WO2007095462, WO2007101060, WO2007105650 beschrieben sind, verabreicht.

Bei einer weiteren Ausführungsform wird die Verbindung der Formel I in Kombination mit antihypoglykämischen Verbindungen, wie sie in WO2007137008 beschrieben sind, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon), DRL-17564, DRF-2593 (Balaglitazon) oder solchen, wie sie in WO2007060992, WO2007100027, WO2007103252, WO2007122970 beschrieben sind, verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.
Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten bzw. gemischten PPAR alpha/PPAR delta Agonisten, wie z.B. GW9578, GW-590735, K-111, LY-674, KRP-101, DRF-10945, LY-518674, CP-900691, BMS-687453, BMS-711939 oder solchen wie sie in WO2001040207, WO2002096894, WO2005097076, WO2007056771, WO2007087448, WO2007089667, WO2007089557, WO2007102515, WO2007103252, JP2007246474, WO2007118963, WO2007118964, WO2007126043 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat), MBX-213 oder wie in WO 00/64888, WO 00/64876, WO03/020269, WO2007099553, US2007276041, WO2007085135, WO2007085136 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178, WO2007071766, WO2007101864, US2007244094, WO2007119887 beschrieben sind, verabreicht.

Bei einer Ausführungsform der Erfindung wird die Verbindung der Formel I in Kombination mit einem pan-SPPARM (selective PPAR modulator alpha, gamma, delta), wie z.B. GFT-505, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht.

Bei einer Ausführungsform wird die Verbindung der Formel I in Kombination mit einem α-Glukosidase-Inhibitor, wie z.B. Miglitol oder Acarbose oder solchen, wie sie z.B. in WO2007114532, WO2007140230 beschrieben sind, verabreicht.
Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit GlukagonRezeptor-Antagonisten, wie z.B. A-770077 oder NNC-25-2504 oder wie in WO2004100875, WO2005065680, WO2006086488, WO2007106181, WO2007111864, WO2007120270, WO2007120284, WO2007123581, WO2007136577 beschrieben, verabreicht.
Bei einer weiteren Ausführungsform wird die Verbindung der Formel I in Kombination mit einer Antisense-Verbindung, z.B. ISIS-325568, verabreicht, welche die Produktion des Glukagonrezeptors inhibiert.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847, WO2007061923, WO2007075847, WO2007089512, WO2007104034, WO2007117381, WO2007122482, WO2007125103, WO2007125105, US2007281942 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515, WO2006104030, WO2007014619, WO2007137962 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200, GW-825964X, KRP-104, DP-893, ABT-341, ABT-279 oder ein anderes Salz davon, S-40010, S-40755, PF-00734200, BI-1356, PHX-1149, Alogliptin oder solchen Verbindungen wie sie in WO2003074500, WO2003106456, WO2004037169, WO200450658, WO2005037828, WO2005058901, WO2005012312, WO2005/012308, WO2006039325, WO2006058064, WO2006015691, WO2006015701, WO2006015699, WO2006015700, WO2006018117, WO2006099943, WO2006099941, JP2006160733, WO2006071752, WO2006065826, WO2006078676, WO2006073167, WO2006068163, WO2006085685, WO2006090915, WO2006104356, WO2006127530, WO2006111261, WO2007015767 (LY-2463665), WO2007024993, WO2007029086, WO2007063928, WO2007070434, WO2007071738, WO2007077508, WO2007087231, WO2007097931, WO2007099385, WO2007100374, WO2007112347, WO2007112669, WO2007113226, WO2007113634, WO2007115821, WO2007116092, US2007259900, EP1852108, US2007270492, WO2007126745, WO2007136603 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Eucreas^{(R)}, einer festen Kombination von Vildagliptin mit Metformin Hydrochlorid, verabreicht.

Bei einer Ausführungsform wird die Verbindung der Formel I in Kombination mit einer Kombination eines DPP-IV-Inhibitors mit omega-3-Fettsäuren oder omega-3-Fettsäureestern, wie z.B. in WO2007128801 beschrieben, verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268, SAR 7226, SGL-5083, SGL-5085, SGL-5094, ISIS-388626, Sergliflozin oder Dapagliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895, WO2007080170, WO2007093610, WO2007126117, WO2007128480, WO2007129668, US2007275907, WO2007136116 oder von A. L. Handlon in Expert Opin. Ther. Patents (2005) 15(11), 1531-1540 beschrieben sind, verabreicht.
Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739, DIO-92 ((-)-Ketoconazol) oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005063247, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138508, WO2006138695, WO2006133926, WO2007003521, WO2007007688, US2007066584, WO2007029021, WO2007047625, WO2007051811, WO2007051810, WO2007057768, WO2007058346, WO2007061661, WO2007068330, WO2007070506, WO2007087150, WO2007092435, WO2007089683, WO2007101270, WO2007105753, WO2007107470, WO2007107550, WO2007111921, US2007207985, US2007208001, WO2007115935, WO2007118185, WO2007122411, WO2007124329, WO2007124337, WO2007124254, WO2007127688, WO2007127693, WO2007127704, WO2007127726, WO2007127763, WO2007127765, WO2007127901, US2007270424, JP2007291075, WO2007130898, WO2007135427 beschrieben sind, verabreicht.

Bei einer Ausführungsform wird die Verbindung der Formel I in Kombination mit Inhibitoren der Protein-Tyrosin-Phosphatase-1B (PTP-1B), wie sie z. B. in WO200119830-31, WO200117516, WO2004506446, WO2005012295, WO2005116003, WO2005116003, WO2006007959, DE 10 2004 060542.4, WO2007009911, WO2007028145, WO2007067612-615, WO2007081755, WO2007115058 beschrieben sind, verabreicht.

Bei einer Ausführungsform der Erfindung wird die Verbindung der Formel I in Kombination mit einem Agonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A (Laropiprant) oder MK-0524 oder solchen Verbindungen, wie sie in WO2006045565, WO2006045564, WO2006069242, WO2006085108, WO2006085112, WO2006085113, WO2006124490, WO2006113150, WO2007017261, WO2007017262, WO2007017265, WO2007015744, WO2007027532, WO2007092364, WO2007120575, WO2007134986 beschrieben sind, verabreicht.

Bei einer anderen Ausführungsform der Erfindung wird die Verbindung der Formel I in Kombination mit einer festen Kombination von Niacin mit Simvastatin verabreicht.

Bei einer anderen Ausführungsform der Erfindung wird die Verbindung der Formel I in Kombination mit Nicotinsäure oder "extended release niacin" in Verbindung mit MK-0524A (Laropiprant) verabreicht.

Bei einer weiteren Ausführungsform der Erfindung wird die Verbindung der Formel I in Kombination mit Nicotinsäure oder "extended release niacin" in Verbindung mit MK-0524A (Laropiprant) und mit Simvastatin verabreicht.
Bei einer anderen Ausführungsform der Erfindung wird die Verbindung der Formel I in Kombination mit einem Agonisten des GPR116, wie sie z.B. in WO2006067531, WO2006067532 beschrieben sind, verabreicht.

Bei einer Ausführungsform wird die Verbindung der Formel I in Kombination mit Modulatoren des GPR40, wie sie z.B. in WO2007013689, WO2007033002, WO2007106469, US2007265332, WO2007123225, WO2007131619, WO2007131620, WO2007131621, US2007265332, WO2007131622, WO2007136572 beschrieben sind, verabreicht.

Bei einer Ausführungsform wird die Verbindung der Formel I in Kombination mit Modulatoren des GPR119b wie sie z. B. in WO2004041274 beschrieben sind, verabreicht.

Bei einer Ausführungsform wird die Verbindung der Formel I in Kombination mit Modulatoren des GPR119 (G-Protein-gekoppelter Glukose-abhängiger insulinotroper Rezeptor), wie z.B. PSN-119-1 oder solchen wie sie z. B. in WO2005061489 (PSN-632408), WO2004065380, WO2006018662, WO2007003960-62 und WO2007003964, WO2007116229, WO2007116230 beschrieben sind, verabreicht.

Bei einer weiteren Ausführungsform wird die Verbindung der Formel I in Kombination mit Modulatoren des GPR120, wie sie z.B. in EP1688138 beschrieben sind, verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178, WO2007119837 beschrieben, verabreicht.

Bei einer Ausführungsform wird die Verbindung der Formel I in Kombination mit Inhibitoren der endothelialen Lipase, wie z. B. in WO2007110216 beschrieben, verabreicht.

Bei einer Ausführungsform wird die Verbindung der Formel I in Kombination mit einem Phospholipase A2 Inhibitor wie z.B. Darapladib oder A-002 verabreicht.
Bei einer Ausführungsform wird die Verbindung der Formel I in Kombination mit Myricitrin, einem Lipase-Inhibitor (WO2007119827), verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, WO2005111018, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117, WO2007073117, WO2007083978, WO2007120102, WO2007122634, WO2007125109, WO2007125110 beschrieben.

Bei einer Ausführungsform wird die Verbindung der Formel I in Kombination mit einem

Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Serum/Glucocorticoid regulierten Kinase (SGK), wie z. B. in WO2006072354, WO2007093264 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer weiteren Ausführungsform wird die Verbindung der Formel I in Kombination mit einem Aktivator der AMP-aktivierten Proteinkinase (AMPK), wie sie z. B. in WO2007062568 beschrieben sind, verabreicht.

Bei einer Ausführungsform wird die Verbindung der Formel I in Kombination mit einem Inhibitor der Ceramidkinase, wie sie z. B. in WO2007112914 beschrieben sind, verabreicht.

Bei einer weiteren Ausführungsform wird die Verbindung der Formel I in Kombination mit einem Inhibitor der MAPK-interagierenden Kinase 2 (MNK2), wie sie z.B. in WO2007104053, WO2007115822 beschrieben sind, verabreicht.
Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022057, WO2004022553, WO2005097129, WO2005113544, US2007244140 beschrieben sind, verabreicht.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 oder solchen, wie sie in US2007249583 beschrieben sind, verabreicht.
Bei einer weiteren Ausführungsform der Erfindung wird die Verbindung der Formel I in Kombination mit einem Farnesoid X Rezeptor (FXR) Antagonisten, wie z.B. in

WO2007052843, WO2007070796, WO2007092751, JP2007230909, WO2007095174, WO2007140174, WO2007140183 beschrieben, verabreicht.

Bei einer anderen Ausführungsform der Erfindung wird die Verbindung der Formel I in Kombination mit einem Liganden des Leber X Rezeptors (liver X receptor; LXR), wie z.B. in WO2007092965 beschrieben, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit Fibraten, wie z.B. dem Cholinsalz von Fenofibrat (SLV-348), verabreicht.

Bei einer Ausführungsform der Erfindung wird die Verbindung der Formel I in Kombination mit Fibraten, wie z.B. dem Cholinsalz von Fenofibrat und einem HMGCoA Reduktase Inhibitor, wie z.B. Rosuvastatin, verabreicht.

Bei einer weiteren Ausführungsform der Erfindung wird die Verbindung der Formel I in Kombination mit Bezafibrat und Diflunisal verabreicht.

Bei einer weiteren Ausführungsform der Erfindung wird die Verbindung der Formel I in Kombination mit einer festen Kombination von Fenofibrat oder einem Salz davon mit Simvastatin, Rosuvastatin, Fluvastatin, Lovastatin, Cerivastatin, Pravastatin oder Atorvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2002050060, WO2002050068, WO2004000803, WO2004000804, WO2004000805, WO2004087655, WO2004097655, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163, WO2007059871, US2007232688, WO2007126358 beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.
Bei einer Ausführungsform der Erfindung ist der weitere Wirkstoff ein
Diphenylazetidinonderivat, wie z.B. in US 6,992,067 oder US 7,205,290 beschrieben.

Bei einer weiteren Ausführungsform der Erfindung ist der weitere Wirkstoff ein Diphenylazetidinonderivat, wie z.B. in US 6,992,067 oder US 7,205,290 beschrieben, kombiniert mit einem Statin, wie z.B. Simvastatin, Fluvastatin, Pravastatin, Lovastatin, Cerivastatin, Atorvastatin oder Rosuvastatin.

Bei einer Ausführungsform der Erfindung wird die Verbindung der Formel I in Kombination mit einer festen Kombination von Lapaquistat, einem Squalensynthase-Inhibitor, mit Atorvastatin verabreicht.

Bei einer Ausführungsform der Erfindung wird die Verbindung der Formel I in Kombination mit einem CETP-Inhibitor, wie z.B. Torcetrapib, Anacetrapib oder JTT-705 oder solchen wie sie in WO2006002342, WO2006010422, WO2006012093, WO2006073973, WO2006072362, WO2007088996, WO2007088999, US2007185058, US2007185113, US2007185154, US2007185182, WO2006097169, WO2007041494, WO2007090752, WO2007107243, WO2007120621, US2007265252, US2007265304, WO2007128568, WO2007132906 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, WO2007009655-56 beschrieben, verabreicht.

Bei einer Ausführungsform wird die Verbindung der Formel I in Kombination mit Agonisten des GPBAR1 (G-protein-coupled-bile-acid-receptor-1; TGR5), wie sie z.B. in WO2007110237, WO2007127505 beschrieben sind, verabreicht.

Bei einer Ausführungsform der Erfindung wird die Verbindung der Formel I in Kombination mit einem polymeren Gallensäureadsorber, wie z.B. Cholestyramin, Colesevelam Hydrochlorid, verabreicht.
Bei einer Ausführungsform der Erfindung wird die Verbindung der Formel I in Kombination mit einem Phytosterole enthaltenden Kaugummi (Reductol™) verabreicht.

Bei einer Ausführungsform der Erfindung wird die Verbindung der Formel I in Kombination mit einem Inhibitor des mikrosomalen Triglycerid-Transfer-Proteins (MTP-Inhibitor), wie z.B. Implitapide , BMS-201038, R-103757, AS-1552133, SLx-4090, AEGR-733 oder solchen wie in WO2005085226, WO2005121091, WO2006010423, WO2006113910 beschrieben, verabreicht.

Bei einer anderen Ausführungsform der Erfindung wird die Verbindung der Formel I in

Kombination mit einem Antagonisten des Somatostatin 5 Rezeptors (SST5 Rezeptor), wie z.B. solchen wie sie in WO2006094682 beschrieben sind, verabreicht.

Bei einer Ausführungsform der Erfindung wird die Verbindung der Formel I in Kombination mit einem ACAT-Inhibitor, wie z.B. Avasimibe, SMP-797 oder KY-382, verabreicht.

Bei einer weiteren Ausführungsform der Erfindung wird die Verbindung der Formel I in Kombination mit einem Inhibitor der Leber-Carnitin Palmitoyltransferase-1 (L-CPT1), wie sie z.B. in WO2007063012, WO2007096251 (ST-3473) beschrieben sind, verabreicht.

Bei einer Ausführungsform der Erfindung wird die Verbindung der Formel I in Kombination mit einem Squalen Synthetase Inhibitor, wie z.B. BMS-188494, TAK-475 (Lapaquistat Acetat) oder wie in WO2005077907, JP2007022943 beschrieben, verabreicht.

Bei einer Ausführungsform der Erfindung wird die Verbindung der Formel I in Kombination mit ISIS-301012 (Mipomersen), einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit einem Adenosin A2B Rezeptor Agonisten (Adenosin A2B R) wie z.B. ATL-801 verabreicht.

Bei einer anderen Ausführungsform der Erfindung wird die Verbindung der Formel I in Kombination mit einem Modulator der Adenosin A2A und/oder Adenosin A3 Rezeptoren, wie z.B. in WO2007111954, WO2007121918, WO2007121921, WO2007121923 beschrieben, verabreicht.

Bei einer Ausführungsform der Erfindung wird die Verbindung der Formel I in Kombination mit einem Adenosin A2B Rezeptor Antagonisten (Adenosin A2B R), wie sie in US2007270433 beschrieben sind, verabreicht.

Bei einer Ausführungsform wird die Verbindung der Formel I in Kombination mit Hemmstoffen der Acetyl-CoA Carboxylase (ACC1 und/oder ACC2) wie z. B. solchen wie in WO199946262, WO200372197, WO2003072197, WO2005044814, WO2005108370, JP2006131559, WO2007011809, WO2007011811, WO2007013691, WO2007095601-603, WO2007119833 beschrieben, verabreicht.

Bei einer anderen Ausführungsform wird die Verbindung der Formel I in Kombination mit Modulatoren der mikrosomalen Acyl-CoA:Glycerol-3-Phosphat-Acyltransferase 3 (GPAT3, beschrieben in WO2007100789) oder mit Modulatoren der mikrosomalen Acyl-CoA:Glycerol-3-Phosphat-Acyltransferase 4 (GPAT4, beschrieben in WO2007100833) verabreicht.

Bei einer weiteren Ausführungsform wird die Verbindung der Formel I in Kombination mit Modulatoren der Xanthin-Oxidoreductase (XOR) verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolie Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804 oder die Verbindung "NPY-5-BY" der Firma Banyu oder wie sie z. B. in WO2006001318, WO2007103295, WO2007125952 beschrieben sind;
NPY-4-Rezeptorantagonisten wie sie z. B. in WO2007038942 beschrieben sind;
NPY-2-Rezeptorantagonisten wie sie z. B. in WO2007038943 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166 beschrieben sind;
Derivaten des Peptids Obestatin wie sie WO2006096847 beschrieben sind;
CB1R (Cannabinoid Rezeptor 1) Antagonisten (wie z.B. Rimonabant, Surinabant (SR147778), SLV-319, AVE-1625, Taranabant (MK-0364) oder Salze davon, V-24343 oder solche Verbindungen wie sie in z. B. EP 0656354, WO 00/15609, WO2001/64632-64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007018460, WO2007016460, WO2007020502, WO2007026215, WO2007028849, WO2007031720, WO2007031721, WO2007036945, WO2007038045, WO2007039740, US20070015810, WO2007046548, WO2007047737, WO2007057687, WO2007062193, WO2007064272, WO2007079681, WO2007084319, WO2007084450, WO2007086080, EP1816125, US2007213302, WO2007095513, WO2007096764, US2007254863, WO2007119001, WO2007120454, WO2007121687, WO2007123949, US2007259934, WO2007131219, WO2007133820, WO2007136607, WO2007136571, US7297710, WO2007138050, WO2007140385, WO2007140439 beschrieben sind);
Cannabinoid Rezeptor 1 / Cannabinoid Rezeptor 2 (CB1/CB2) modulierende Verbindungen wie z.B. delta-9-Tetrahydrocannabivarin oder solchen wie sie z.B. in WO2007001939, WO2007044215, WO2007047737, WO2007095513, WO2007096764, WO2007112399, WO2007112402 beschrieben sind;
Modulatoren der FAAH (fatty acid amide hydrolase) wie sie z.B. in WO2007140005 beschrieben sind;
Vanilloid-1-Rezeptor Modulatoren (Modulatoren des TRPV1), wie sie z.B. in WO2007091948, WO2007129188, WO2007133637 beschrieben sind;
Aktivatoren des Capsaicinrezeptors, wie sie z.B. in JP2007210969 beschrieben sind;
Agonisten des Prostaglandinrezeptors, wie z.B. Bimatoprost oder solchen Verbindungen wie sie in WO2007111806 beschrieben sind;
MC4-Rezeptor Agonisten (Melanocortin-4 Rezeptor Agonisten, MC4R Agonisten wie z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlor-phenyl)-2-oxo-ethyl]-amid; (WO 01/91752)) oder LB53280, LB53279, LB53278 oder THIQ, MB243, RY764, CHIR-785, PT-141, MK-0493 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO2004072077, WO2006021655-57, WO2007009894, WO2007015162, WO2007041061, WO2007041052, JP2007131570, EP-1842846, WO2007096186, WO2007096763 beschrieben sind;
Orexin-Rezeptor 1 Antagonisten (OX1R Antagonisten), Orexin-Rezeptor 2 Antagonisten (OX2R Antagonisten) oder gemischte OX1R/OX2R Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224, WO2007085718, WO2007088276, WO2007116374;WO2007122591, WO2007126934, WO2007126935 beschrieben sind);
Histamin H3 Rezeptor Antagonisten/inverse Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, US2005171181 (z.B. PF-00389027), WO2006107661, WO2007003804, WO2007016496, WO2007020213, WO2007049798, WO2007055418, WO2007057329, WO2007065820, WO2007068620, WO2007068641, WO2007075629, WO2007080140, WO2007082840, WO2007088450, WO2007088462, WO2007094962, WO2007099423, WO2007100990, WO2007105053, WO2007106349, WO2007110364, WO2007115938, WO2007131907, WO2007133561, US2007270440, WO2007135111 beschrieben sind);
Histamin H1 / Histamin H3 Modulatoren, wie z. B. Betahistin bzw. seinem Dihydrochlorid;
CRF-Antagonisten (z.B. [2-Methyl-9-(2,4,6-trimethyl-phenyl)-9H-1,3,9-triaza-fluoren-4-yl]-dipropyl-amin (WO 00/66585) oder solche CRF1-Antagonisten, wie sie in WO2007105113, WO2007133756 beschrieben sind);
CRF BP-Antagonisten (z.B. Urocortin);
Urocortin-Agonisten;
Agonisten des beta-3 Adrenoceptors wie z.B. 1-(4-Chlor-3-methansulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2002038544, WO2007048840-843 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649, WO2007092416; WO2007093363-366, WO2007114902, WO2007114916 beschrieben sind);
CCK-A (CCK-1) Agonisten (wie z.B. {2-[4-(4-Chlor-2,5-dimethoxy-phenyl)-5-(2-cyclohexylethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034, WO2007120655, WO2007120688, WO2007120718 beschrieben sind;
Serotonin-Wiederaufnahme-Inhibitoren (z.B. Dexfenfluramine);
gemischte Serotonin-/Dopamin-Wiederaufnahme-Inhibitoren (z.B. Bupropion) oder feste Kombinationen von Bupropion mit Naltrexon oder Bupropion mit Zonisamid;
gemischte Wiederaufnahmeinhibitoren wie z.B. DOV-21947;
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
gemischte Dopamin/Norepinephrin/Acetylcholin-Wiederaufnahme-Inhibitoren (z.B. Tesofensine) oder solchen wie sie z.B. in WO2006085118 beschrieben sind;
5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006004937, US2006025601, WO2006028961, WO2006077025, WO2006103511, WO2007028132, WO2007084622, US2007249709; WO2007132841, WO2007140213 beschrieben sind);
5-HT6 Rezeptor Modulatoren, wie z.B. E-6837, BVT-74316 oder PRX-07034 oder solche wie sie z.B. in WO2005058858, WO2007054257, WO2007107373, WO2007108569, WO2007108742-744 beschrieben sind;
Agonisten des Estrogenrezeptors gamma (ERR Agonisten), wie sie z.B. in WO2007131005 beschrieben sind;
Sigma-1 Rezeptorantagonisten, wie sie z.B. in WO2007098953, WO2007098961 beschrieben sind;
Muscarin 3 Rezeptor (M3R) Antagonisten, wie sie z.B. in WO2007110782 beschrieben sind;
Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734, WO2007127457 beschrieben sind;
Growth Hormone Secretagogue Receptor Modulatoren wie z.B. JMV-2959, JMV-3002, JMV-2810, JMV-2951 oder solchen, wie sie in WO2006012577 (z.B. YIL-781 oder YIL-870), WO2007079239 beschrieben sind;
TRH-Agonisten (siehe z.B. EP 0 462 884);
entkoppelnde Protein 2- oder 3-Modulatoren; Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
Dopaminagonisten (DA-Agonisten, z.B. Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538, WO2007060140, JP2007131584, WO2007071966, WO2007126957, WO2007137103, WO2007137107, WO2007138304, WO2007138311 beschrieben;
Inhibitoren der Fettsäuresynthase (FAS) wie z.B. C75 oder solchen, wie in WO2004005277 beschrieben;
Inhibitoren der Stearoyl-CoA delta9 Desaturase (SCD1) wie sie z.B. in WO2007009236, WO2007044085, WO2007046867, WO2007046868, WO20070501124, WO2007056846, WO2007071023, WO2007130075, WO2007134457, WO2007136746 beschrieben sind;
Inhibitoren des "Adipocyte fatty acid-binding protein aP2" wie z.B. BMS-309403;
Aktivatoren der Adiponectinsekretion, wie z.B. in WO2006082978 beschrieben;
Promotoren der Adiponectinproduktion, wie z.B. in WO2007125946 beschrieben;
Oxyntomodulin;
Oleoyl-Estron
oder Agonisten oder partiellen Agonisten des Schilddrüsenhormonrezeptors (thyroid hormone receptor agonists) wie z. B: KB-2115 oder DITPA oder solche, wie in WO20058279, WO200172692, WO200194293, WO2003084915, WO2004018421, WO2005092316, WO2007003419, WO2007009913, WO2007039125, WO2007110225, WO2007110226, WO2007128492, WO2007132475, WO2007134864 beschrieben
oder Agonisten des Schilddrüsenhormonrezeptors beta (TR-beta) wie z. B. MB-07811 oder
MB-07344, verabreicht.

Bei einer Ausführungsform der Erfindung wird die Verbindung der Formel I in Kombination mit einem Inhibitor der Site-1 Protease (S1P), wie z.B. PF-429242, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® oder Lovaza™ (Omega-3-Fettsäureester; hochkonzentrierte Ethylester der Eicosapentaensäure und der Docosahexaensäure) verabreicht.

Bei einer Ausführungsform wird die Verbindung der Formel I in Kombination mit Lycopin verabreicht.

Bei einer Ausführungsform der Erfindung wird die Verbindung der Formel I in Kombination mit einem Antioxidans, wie z.B. OPC-14117, Succinobucol, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B 12 verabreicht.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer anderen Ausführungsform wird die Verbindung der Formel I in Kombination mit einem Inhibitor der Carboanhydrase Typ 2 (Carbonic anhydrase type 2), wie z.B. solchen, wie in WO2007065948 beschrieben, verabreicht.

Bei einer anderen Ausführungsform wird die Verbindung der Formel I in Kombination mit Topiramat verabreicht.

Bei einer weiteren Ausführungsform wird die Verbindung der Formel I in Kombination mit einer festen Kombination von Topiramat mit Phentermin (Qnexa™) verabreicht.

Bei einer weiteren Ausführungsform wird die Verbindung der Formel I in Kombination mit einer Antisense-Verbindung, z.B. ISIS-377131, verabreicht, welche die Produktion des Glukokortikoidrezeptors inhibiert.

Bei einer Ausführungsform wird die Verbindung der Formel I in Kombination mit einem Agonisten des RUP3 Rezeptors, wie z. B. in WO2007035355 beschrieben, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Tau-Protein-Kinase-1-Inhibitor (TPK1 Inhibitor), wie z. B. in WO2007119463 beschrieben, verabreicht.

Bei einer Ausführungsform wird die Verbindung der Formel I in Kombination mit einem "c-Jun N-terminal kinase" Inhibitor (JNK-Inhibitor), wie z. B. in WO2007125405 beschrieben, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Modulatoren des Glukokortikoidrezeptors (GR), wie z.B. KB-3305 oder solchen Verbindungen wie sie z. B. in WO2005090336, WO2006071609, WO2006135826, WO2007105766 beschrieben sind, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1 (einer NAD⁺-abhängigen Proteindeacetylase); dieser Wirkstoff kann z.B. Resveratrol in geeigneten Formulierungen sein, oder solche Verbindungen wie sie in WO2007019416 (z.B. SRT-1720) genannt sind.

Bei einer Ausführungsform der Erfindung ist der weitere Wirkstoff DM-71 (N-Acetyl-L-Cystein mit Bethanechol).

Bei einer Ausführungsform wird die Verbindung der Formel I in Kombination mit antihypercholesterolemisch wirkenden Verbindungen, wie sie z.B. in WO2007107587, WO2007111994 beschrieben sind, verabreicht.

Bei einer anderen Ausführungsform wird die Verbindung der Formel I in Kombination mit einem cyclischen Peptidagonisten des VPAC2 Rezeptors, wie sie z.B. in WO2007101146, WO2007133828 beschrieben sind, verabreicht.

Bei einer weiteren Ausführungsform wird die Verbindung der Formel I in Kombination mit einem Agonisten des Endothelinrezeptors, wie sie z.B. in WO2007112069 beschrieben sind, verabreicht.

Bei einer weiteren Ausführungsform wird die Verbindung der Formel I in Kombination mit AKP-020 (Bis(ethylmaltolato)oxovanadium-IV) verabreicht.

Bei einer anderen Ausführungsform wird die Verbindung der Formel I in Kombination mit gewebe-selektiven Androgenrezeptor Modulatoren ("tissue-selective androgen receptor modulators"; SARM), wie sie z.B. in WO2007099200 beschrieben sind, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer anderen Ausführungsform der Erfindung ist der weitere Wirkstoff Metreleptin (rekombinantes Methionyl-Leptin) kombiniert mit Pramlintide.

Bei einer weiteren Ausführungsform der Erfindung ist der weitere Wirkstoff das Tetrapeptid ISF-402.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer weiteren Ausführungsform ist der weitere Wirkstoff Geniposidinsäure (geniposidic acid; WO2007100104).

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties & Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

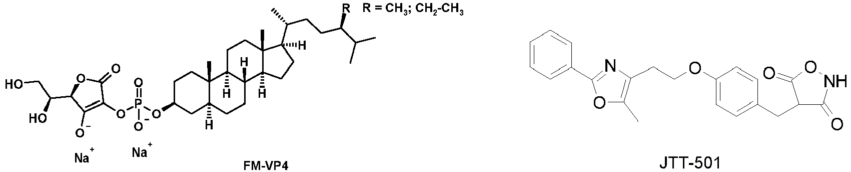

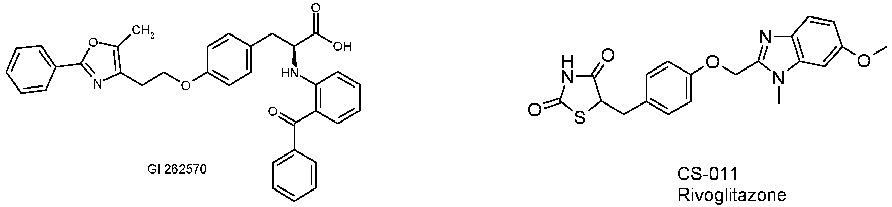

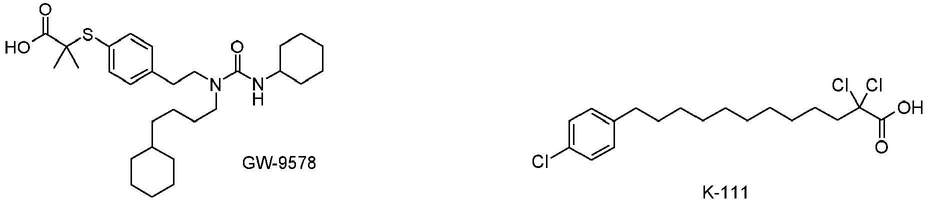

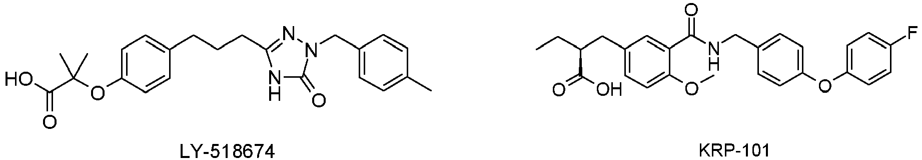

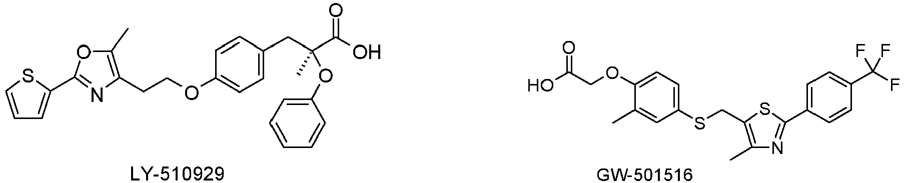

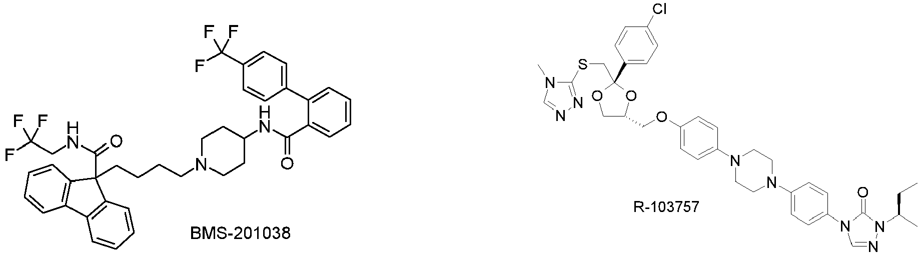

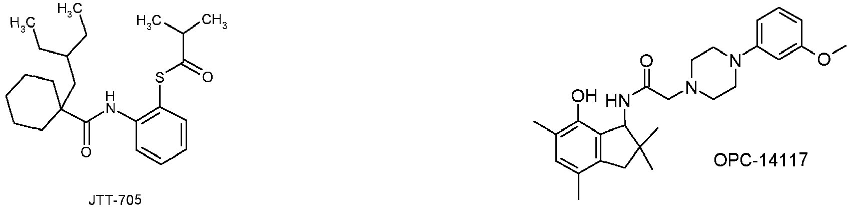

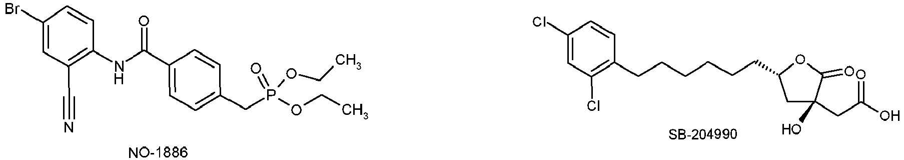

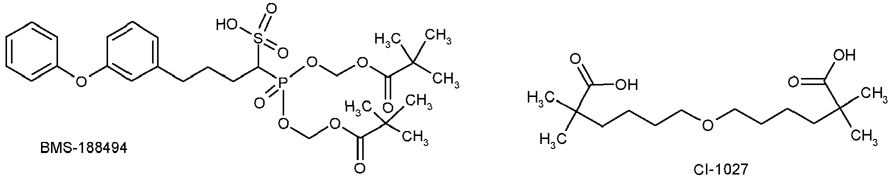

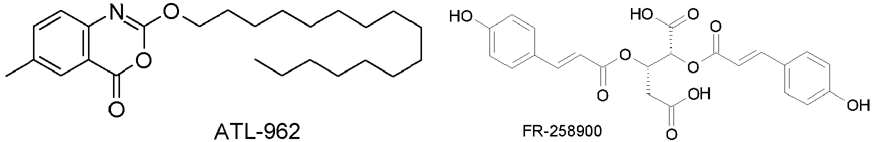

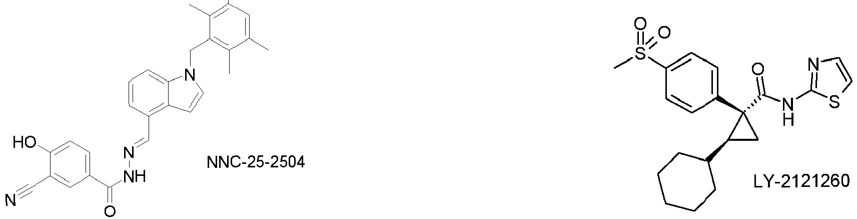

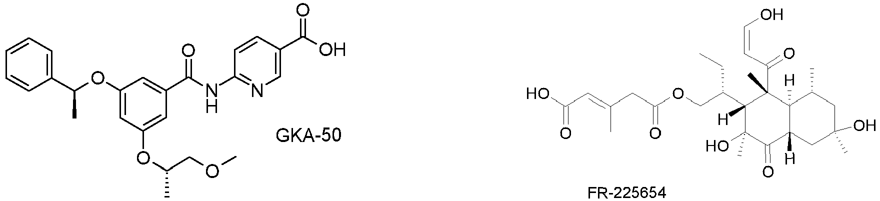

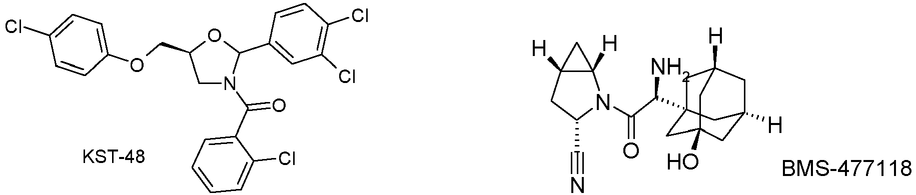

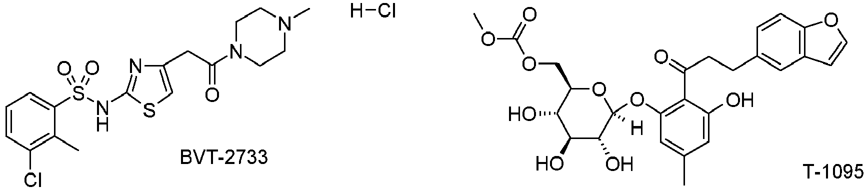

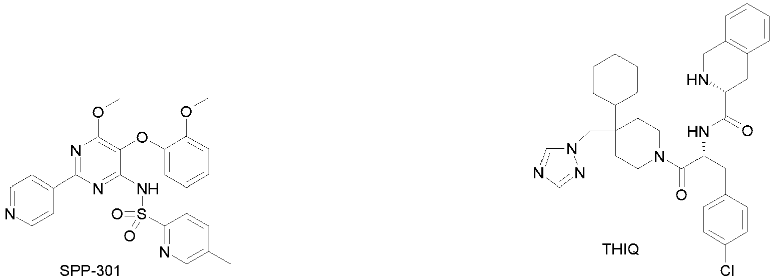

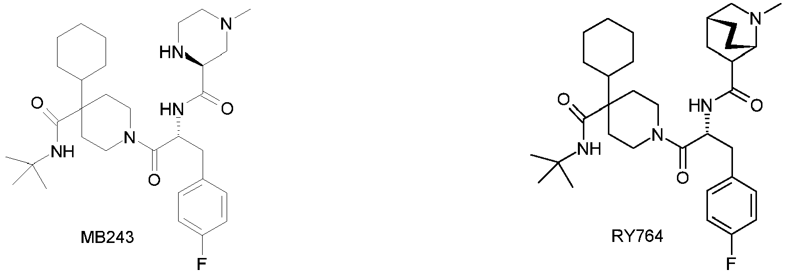

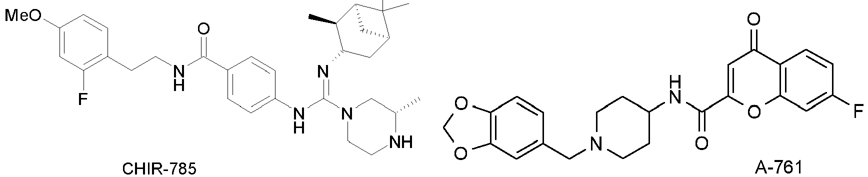

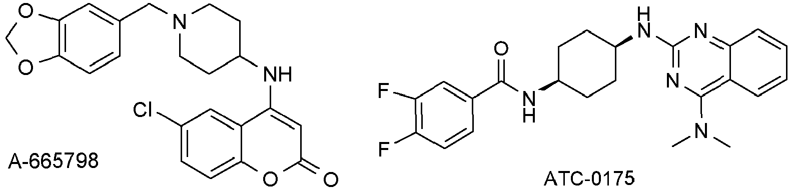

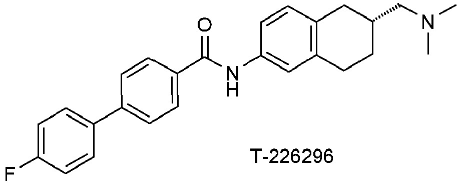

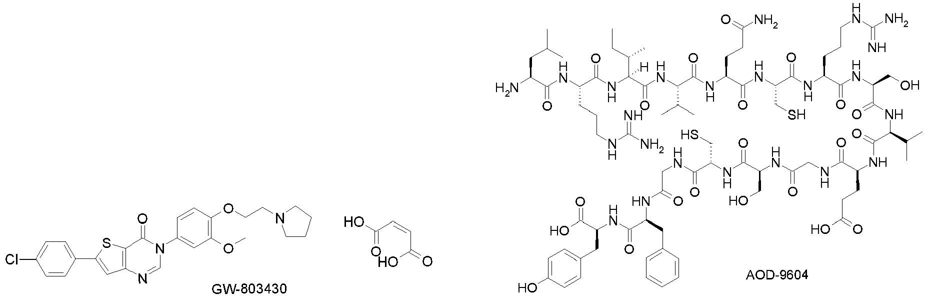

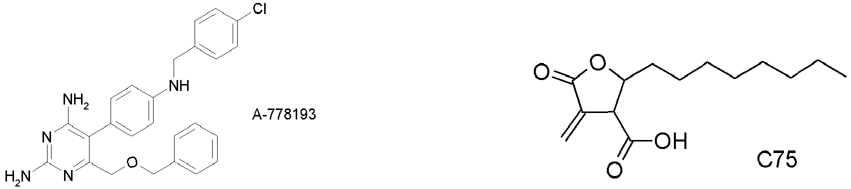

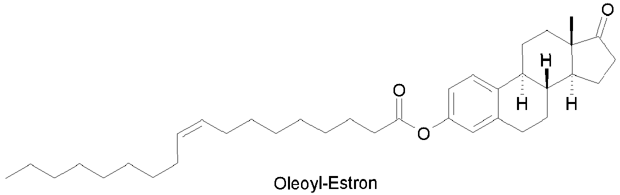

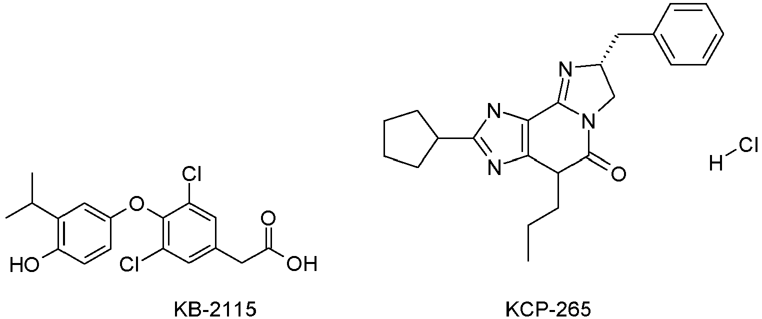

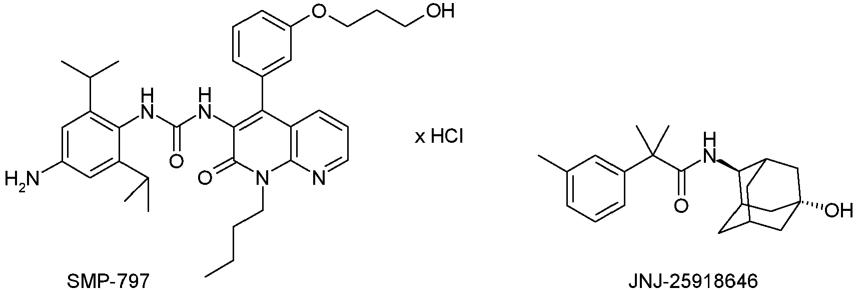

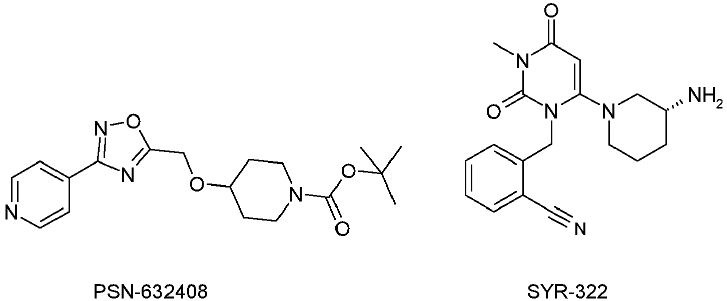

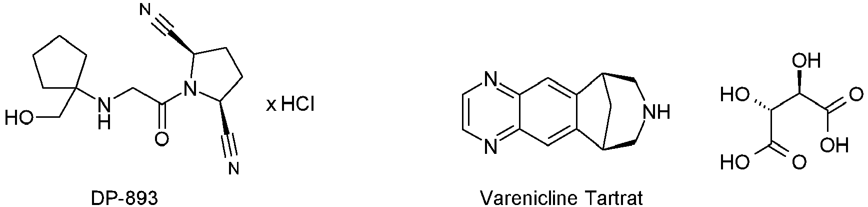

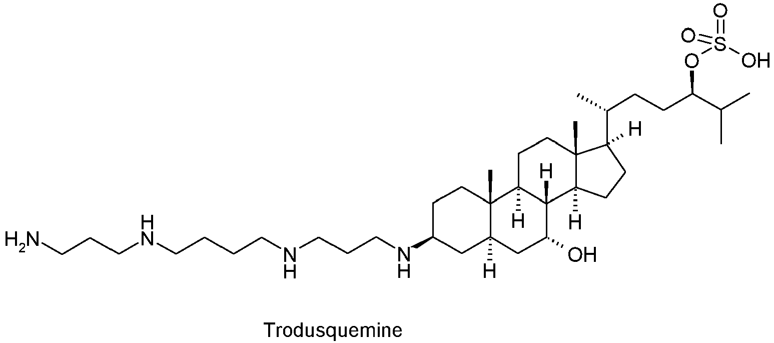

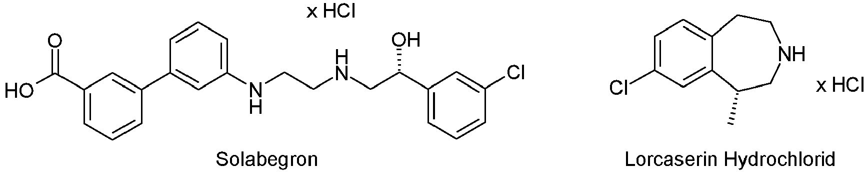

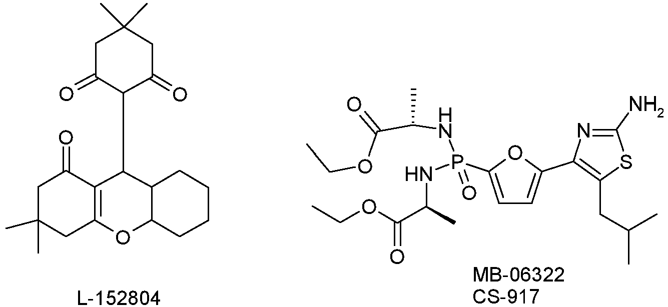

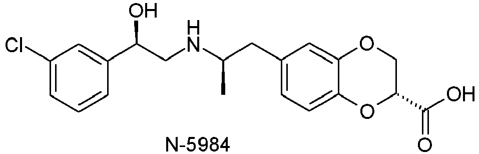

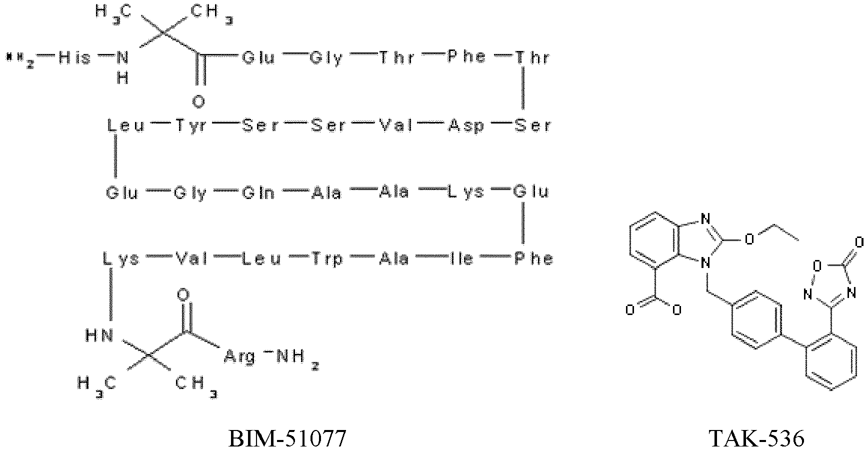

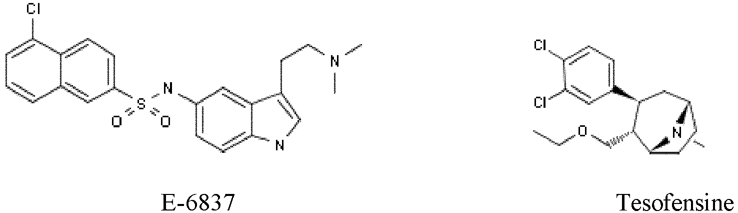

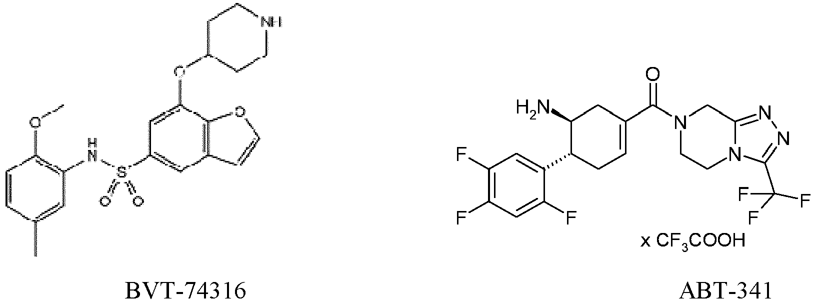

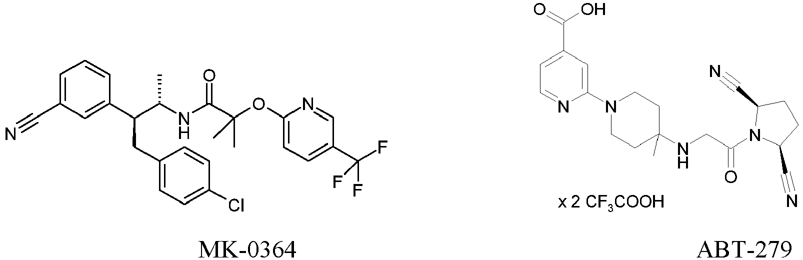

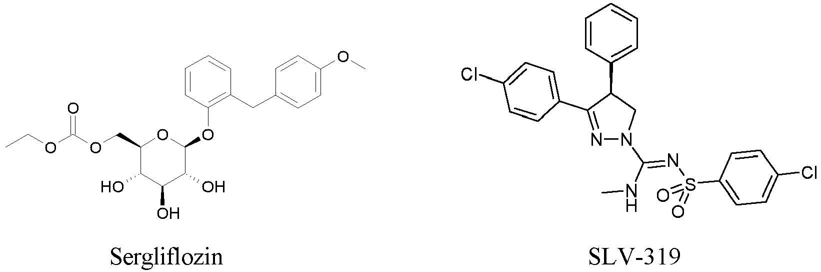

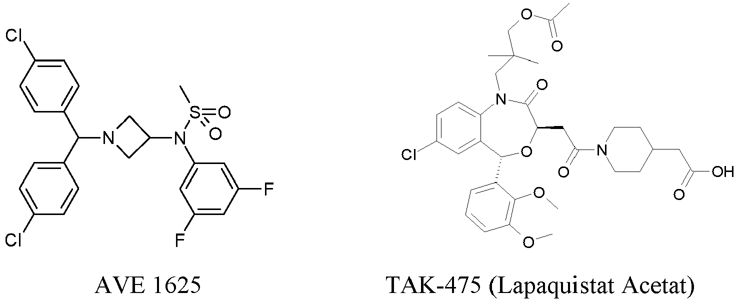

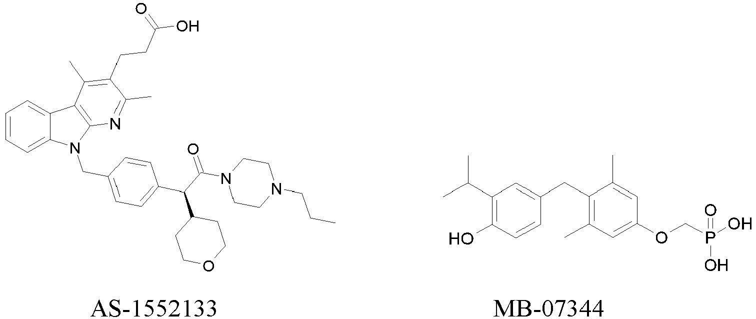

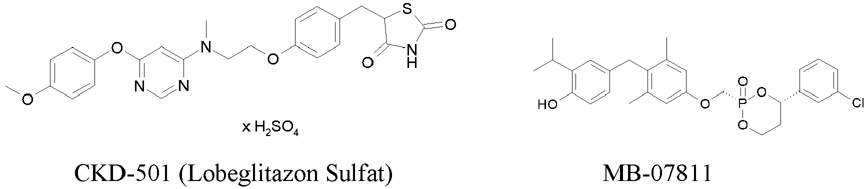

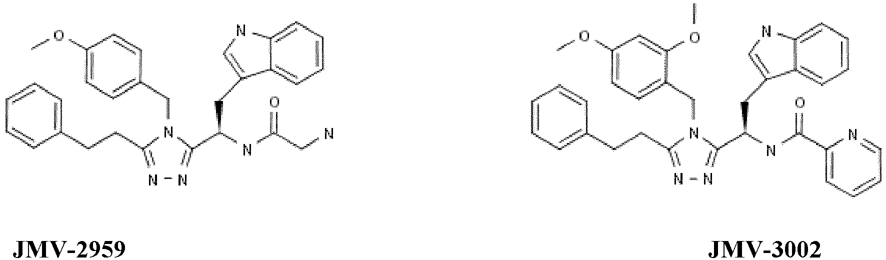

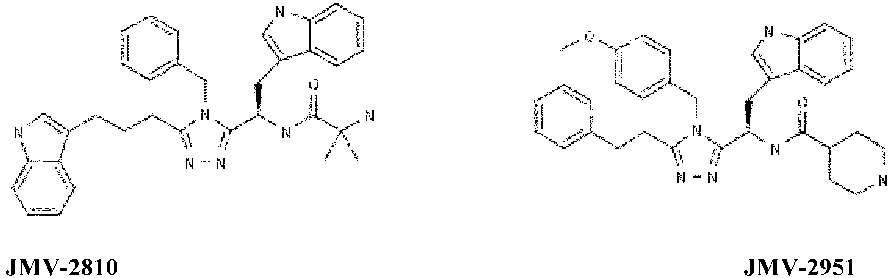

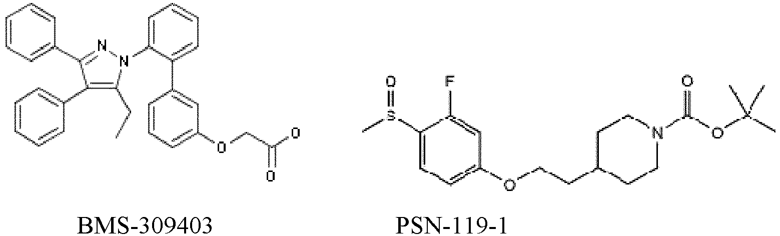

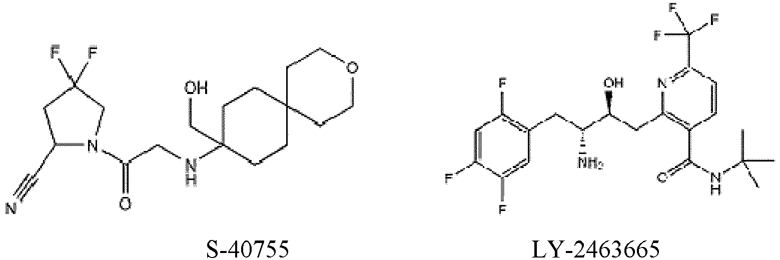

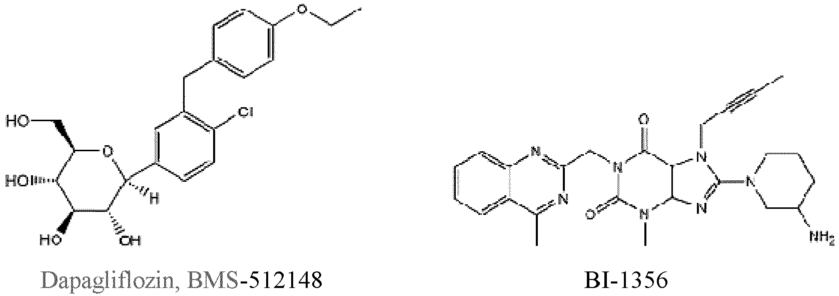

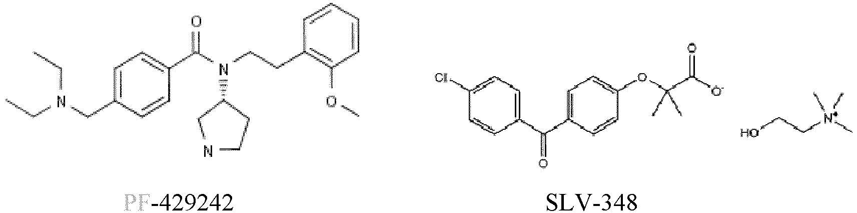

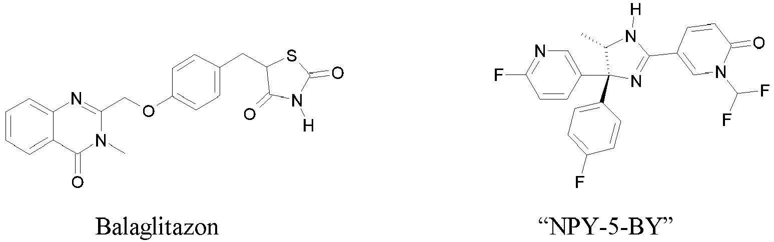

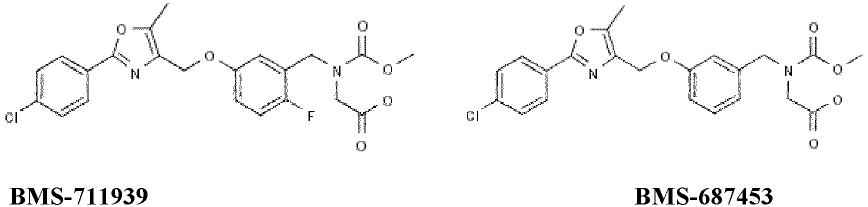

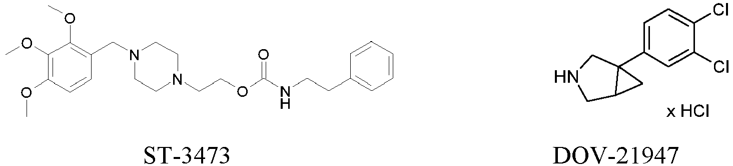

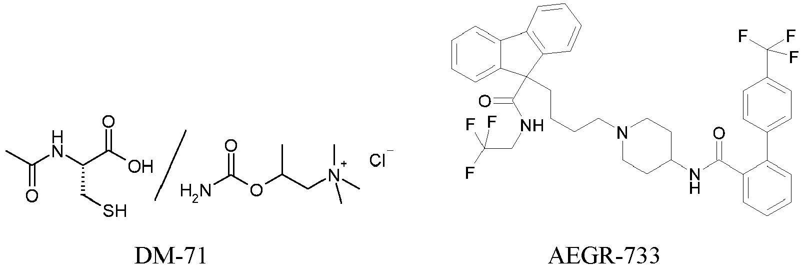

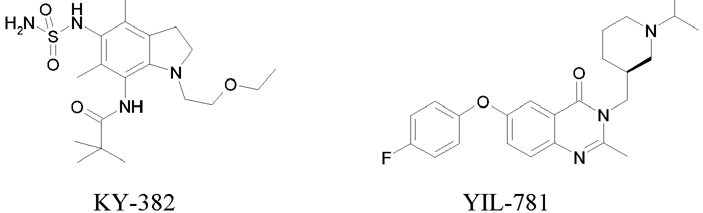

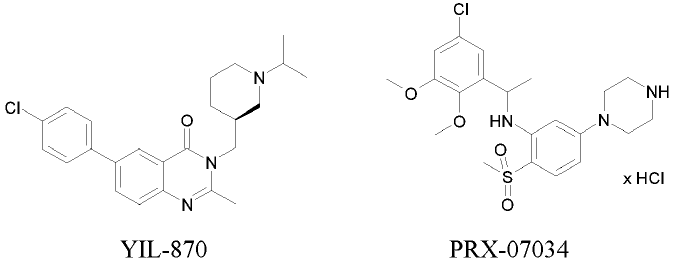

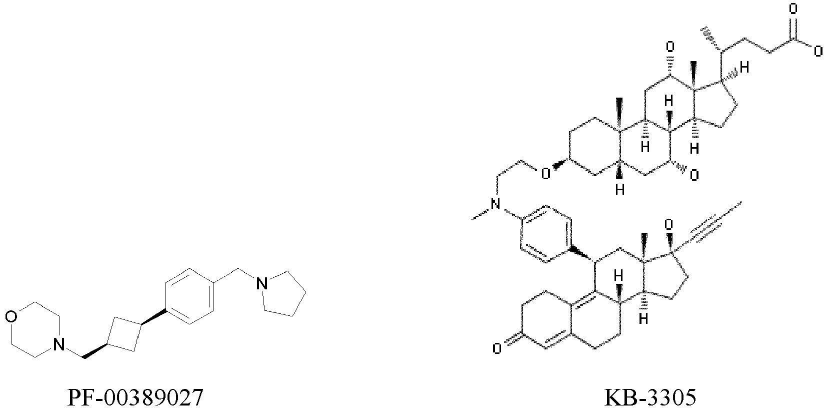

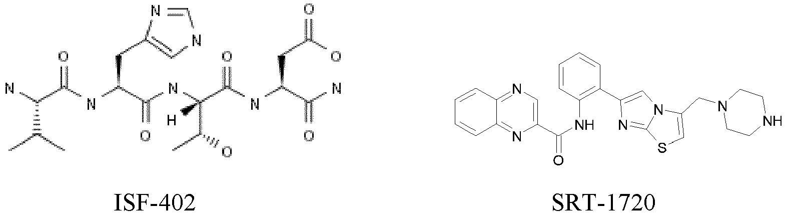

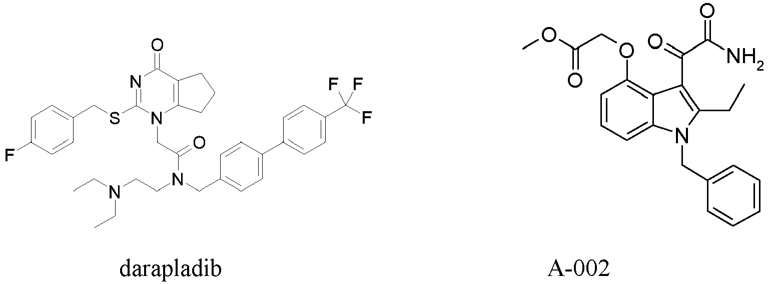

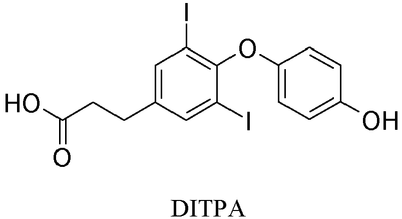

### Beispiele

Die nachfolgend aufgeführten Beispiele und Herstellungsmethoden dienen zur Erläuterung der Erfindung, ohne diese jedoch einzuschränken.

Die erfindungsgemäßen Verbindungen der Formel I können mit Hilfe von im Prinzip bekannten Reaktionen hergestellt werden. Beispielsweise wurden die Verbindungen nach folgenden allgemeinen Reaktionsschemata erhalten.

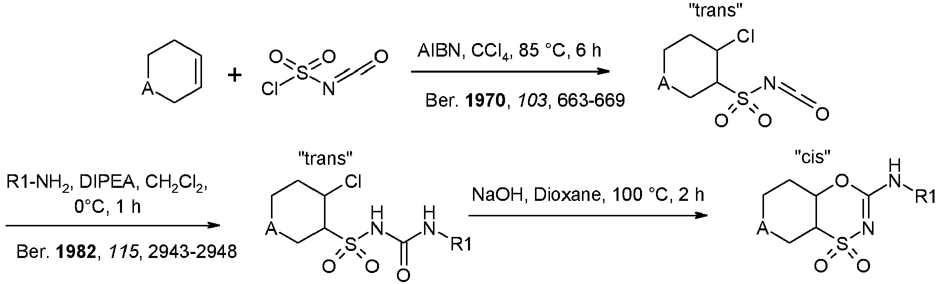

Aus einem cyclischen Alken kann ein entsprechendes Chlorsulfonylisocyanat in einer radikalischen Reaktion hergestellt werden. Dabei wird ausschließlich die cis-Konfiguration erhalten. Anschließend wird mit primären Aminen zu Chlorsulfonylharnstoffen umgesetzt. Die entsprechenden Natriumsalze zyklisieren in der Wärme zu cis-4,4-Dioxo-oxathiazinen.

In den Fällen, in denen während der Synthese Diastereomere oder Racemate entstehen, können diese durch präparative HPLC voneinander getrennt wreden.

Einige der eingesetzten primären Amine sind kommerziell erhältlich. Andere primäre Amine wurden hergestellt wie im folgenden Schema beispielhaft skizziert ist.

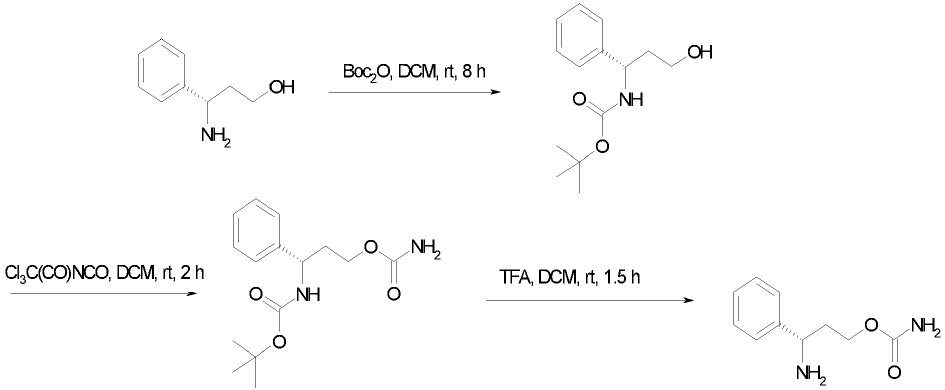

Andere der verwendeten primären Amine wurden hergestellt wie im folgenden Schema beispielhaft skizziert ist.

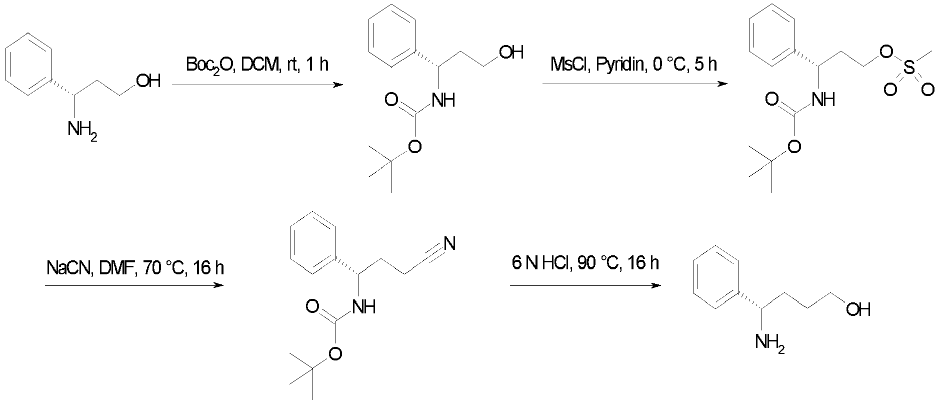

Andere der verwendeten primären Amine wurden hergestellt wie im folgenden Schema beispielhaft skizziert ist.

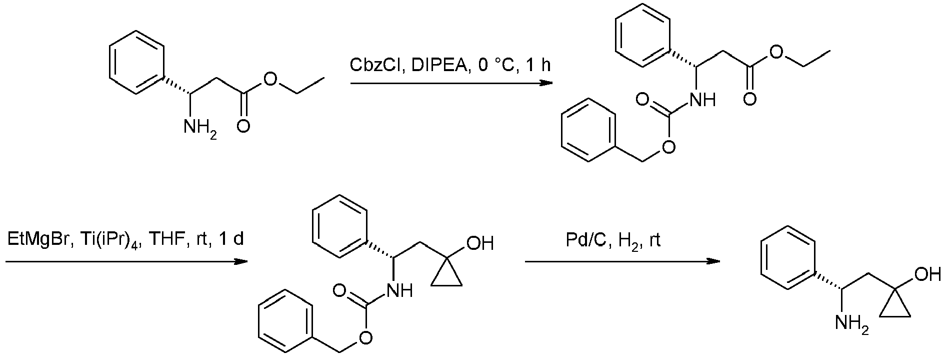

Andere der verwendeten primären Amine wurden hergestellt wie im folgenden Schema beispielhaft skizziert ist.

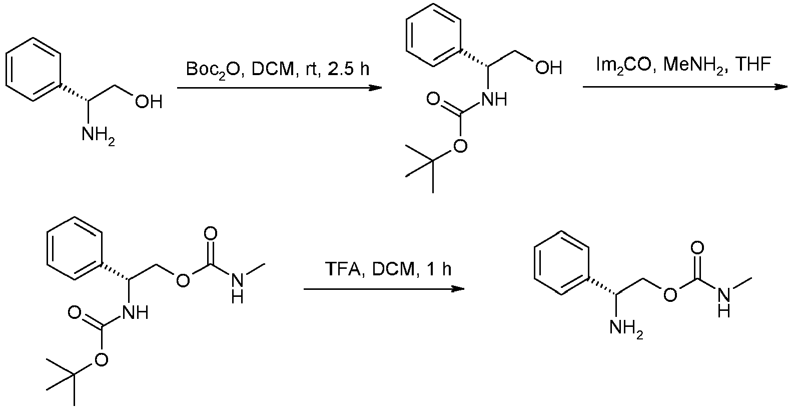

Andere der verwendeten primären Amine wurden hergestellt wie im folgenden Schema beispielhaft skizziert ist.

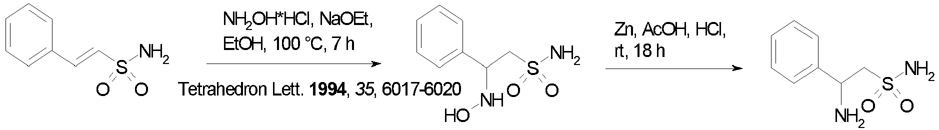

Andere erfindungsgemäße Verbindungen können auf weiteren Wegen erhalten werden, die im folgenden Schema beispielhaft skizziert sind.

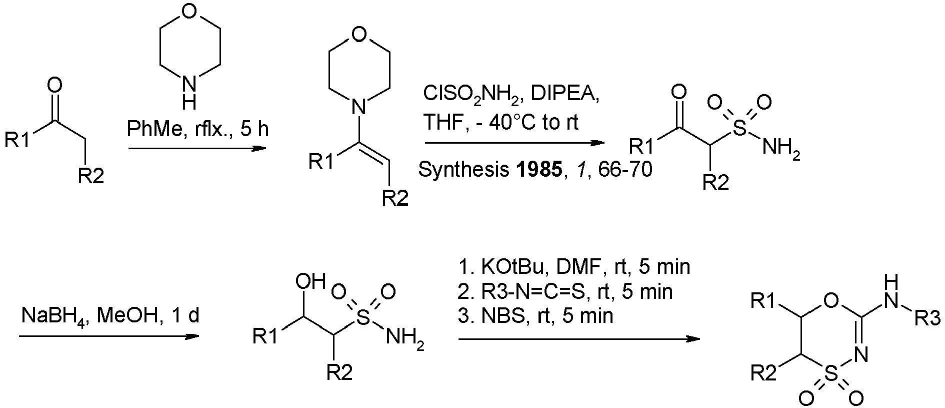

Ein Keton wird mit Morpholin zum Enamin umgesetzt. Dieses wird dann mit Sulfamoylchlorid behandelt. Das entstehende Ketosulfonamid kann mit einem geeigneten Reduktionsmittel, z.B. Natriumborhydrid, zum Hydroxysulfonamid reduziert werden. Dabei kommt es zur Bildung von Diastereomeren und im Falle von cyclischen Ketonen zu cis/trans Isomeren, die voneinander getrennt werden können. Das Verhältnis der Diastereomeren bzw. cis/trans Isomeren ist abhängig von der Natur der Reste R1 und R2 sowie von der Wahl des Reduktionsmittels. Die Behandlung des Hydroxysulfonamids mit Base und einem Isothiocyanat sowie nachfolgendem oxidativen Ringschluss mit NBS liefert die gewünschten 4,4-dioxo-oxathiazine.

Die verwendeten Isothiocyanate werden durch die Reaktion eines primären Amins mit Thiocarbonyldiimidazol erhalten, wobei eventuell vorhandene störende funktionelle Gruppen, z.B. Hydroxylgruppen mit geeigneten Schutzgruppen, z.B. Silylether blockiert wurden. Die Schutzgruppen werden am Ende der Sequenz durch geeignete Verfahren entfernt, z.B. Silylgruppen durch Behandlung mit methanolischer Salzsäure.

In den Fällen, in denen während der Synthese Diastereomere oder Racemate entstehen, können diese durch präparative HPLC voneinander getrennt werden.

Andere erfindungsgemäße Verbindungen können auf weiteren Wegen erhalten werden, die im folgenden Schema beispielhaft skizziert sind.

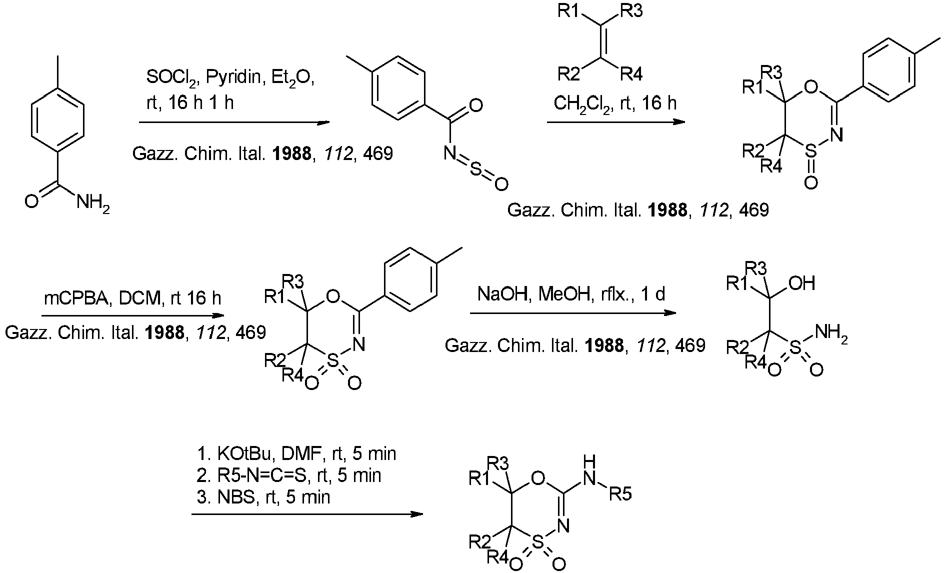

4-Tolylamid wird mit Thionylchlorid und Pyridin zum N-Sulfinyl-4-tolylamid umgesetzt. Dieses reagiert in einer Hetero-Diels-Alder Reaktion mit einem Alken zum 2-Tolyl-4-oxathiazin. Bei cyclischen Alkenen wird dabei ausschließlich die *cis*-Konfiguration erhalten. Nach Oxidation zum 2-Tolyl-4,4-dioxathiazin mit 3-Chlorperbenzoesäure wird mit Natronlauge zum 1,2-Dimethyl-2-hydroxycyclohexylsulfonamid hydrolysiert. Die weitere Behandlung mit Base und einem Isothiocyanat sowie nachfolgendem oxidativen Ringschluss mit NBS liefert die gewünschten 4,4-Dioxo-oxathiazine.

In den Fällen, in denen während der Synthese Diastereomere oder Racemate entstehen, können diese durch präparative HPLC voneinander getrennt werden.

Wieder andere Beispiele wurden erhalten wie im folgenden Schema angedeutet.

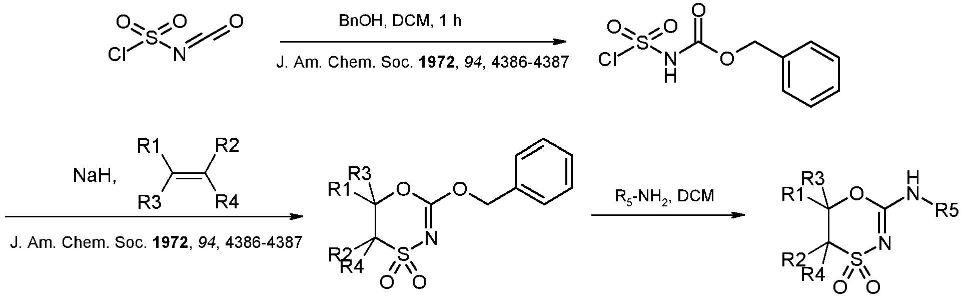

Chlorsulfonylisocyanat wird zunächst mit Benzylalkohol zum entsprechenden Chlorsulfonylcarbamat umgesetzt. Dieses reagiert in einer 2+4-Cycloaddition mit einem Alken zum entsprechenden 2-(benzyloxy)-4,4-dioxothiazin. Bei cyclischen Alkenen wird dabei ausschließlich die cis-Konfiguration erhalten. Die Reaktion mit Aminen liefert die gewünschten 4,4-Dioxo-oxathiazine.

Sofern die eingesetzten Amine gewünschte funktionelle Gruppen in maskierter Form enthalten, werden diese durch geeignete Verfahren in die gewünschten Funktionalitäten umgewandelt. Beispielsweise kann ein Ester durch die Reaktion mit DIBAL-H und anschließend NaBH₄ in einen Alkohol umgewandelt werden.

In den Fällen, in denen während der Synthese Diastereomere oder Racemate entstehen, können diese durch präparative HPLC voneinander getrennt werden.

Die- nachfolgend aufgeführten Beispiele dienen zur Erläuterung der Erfindung, ohne diese jedoch einzuschränken.

**Tabelle 1:**

| Beispiel | CHEMISTRY | Methode | Retentions zeit | Molgewicht (g/mol) | Name |
|---|---|---|---|---|---|
| 1 | 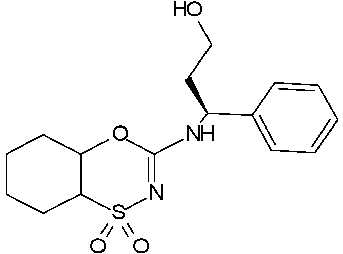 | A | 1,052 | 338,4 | (S)-3-(-1,1-Dioxo-4a,5,6,7,8,8a-hexahydro-1H-1lambda6-benzo[1,4,3]oxathiazin-3-ylamino)-3-phenyl-propan-1-ol |
| 2 | 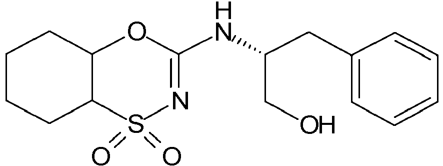 | G | 1,26 | 338,4 | (R)-2-(-1,1-Dioxo-4a,5,6,7,8,8a-hexahydro-1H. 1lambda6-benzo[1,4,3]oxathiazin-3-ylamino)-3-phenyl-propan-1-ol |
| 3 | 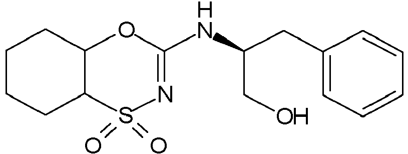 | J | 2,63 | 338,4 | (S)-2-(-1,1-Dioxo-4a,5,6,7,8,8a-hexahydro-1H-1lambda6-benzo[1,4,3]oxathiazin-3-ylamino)-3-phenyl-propan-1-ol |
| 4 | 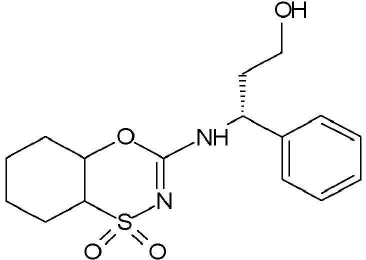 | K | 1,83 | 338,4 | (R)-3-(-1,1-Dioxo-4a,5,6,7,8,8a-hexahydro-1H 1lambda6-benzo[1,4,3]oxathiazin-3-ylamino)-3-phenyl-propan-1-ol |
| 5 | 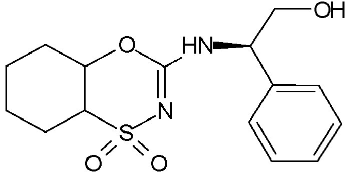 | K | 1,83 | 324,4 | (R)-2-(-1,1-Dioxo-4a,5,6,7,8,8a-hexahydro-1H 1lambda6-benzo[1,4,3]oxathiazin-3-ylamino)-2-phenyl-ethanol |
| 6 | 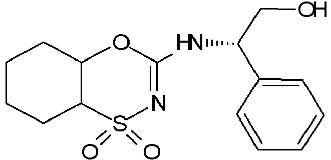 | K | 1,83 | 324,4 | (S)-2-(-1,1-Dioxo-4a,5,6,7,8,8a-hexahydro-1H-1lambda6-benzo[1,4,3]oxathiazin-3-ylamino)-2-phenyl-ethanol |
| 7 | 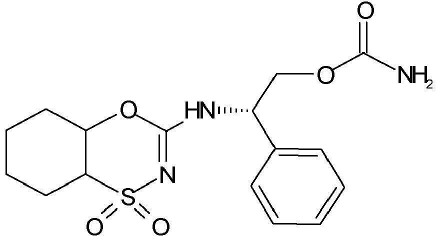 | K | 1,88 | 367,4 | Carbamic acid (S)-2-(-1,1-dioxo-4a,5,6,7,8,8a hexahydro-1H-1lambda6-benzo[1,4,3]oxathiazin-3-ylamino)-2-phenyl-ethyl ester |
| 8 | 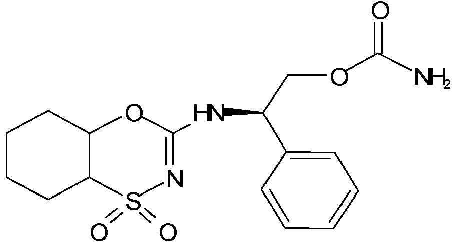 | K | 1,88 | 367,4 | Carbamic acid (R)-2-(-1,1-dioxo-4a,5,6,7,8,8a hexahydro-1H-1lambda6-benzo[1,4,3]oxathiazin-3-ylamino)-2-phenyl-ethyl ester |
| 9 | 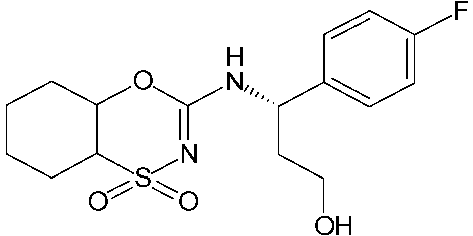 | A | 1,115 | 356,4 | (S)-3-(-1,1-Dioxo-4a,5,6,7,8,8a-hexahydro-1H 1lambda6-benzo[1,4,3]oxathiazin-3-ylamino)-3-(4-fluoro-phenyl)-propan-1-ol |
| 10 | 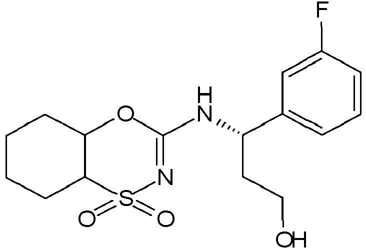 | A | 1,116 | 356,4 | (S)-3-(-1,1-Dioxo-4a,5,6,7,8,8a-hexahydro-1H-1lambda6-benzo[1,4,3]oxathiazin-3-ylamino)-3-(3-fluoro-phenyl)-propan-1-ol |
| 11 | 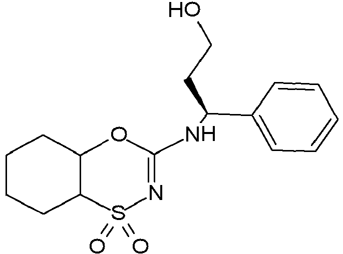 | D | 4,572 | 338,4 | (-)-(S)-3-(1,1-Dioxo-4a,5,6,7,8,8a-hexahydro-1H-1lambda6-benzo[1,4,3]oxathiazin-3-ylamino)-3-phenyl-propan-1-ol * |
| 12 | 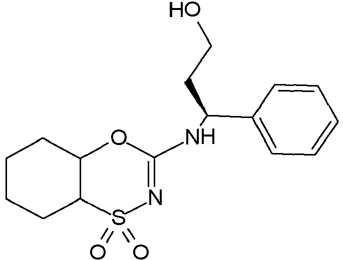 | D | 6,813 | 338,4 | (-)-(S)-3-(1,1-Dioxo-4a,5,6,7,8,8a-hexahydro-1H-1lambda6-benzo[1,4,3]oxathiazin-3-ylamino)-3-phenyl-propan-1-ol * |
| 13 | 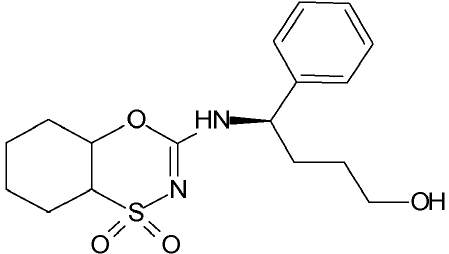 | K | 1,89 | 352,4 | (R)-4-(-1,1 -Dioxo-4a,5,6,7,8,8a-hexahydro-1H-1lambda6-benzo[1,4,3]oxathiazin-3-ylamino)-4-phenyl-butan-1-ol |
| 14 | 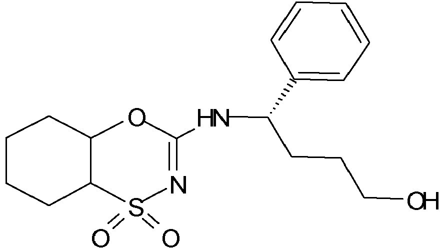 | K | 1,88 | 352,4 | (S)-4-(-1,1-Dioxo-4a,5,6,7,8,8a-hexahydro-1H-1lambda6-benzo[1,4,3]oxathiazin-3-ylamino)-4-phenyl-butan-1-ol |
| 15 | 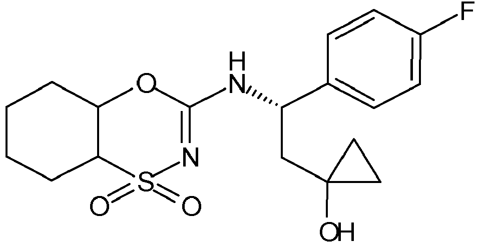 | I | 6,139 | 382,4 | 1-[(S)-2-(1,1-Dioxo-4a,5,6,7,8,8a-hexahydro-1H-1lambda6-benzo[1,4,3]oxathiazin-3-ylamino)-2-(4-fluoro-phenyl)-ethyl]-cyclopropanol * |
| 16 | 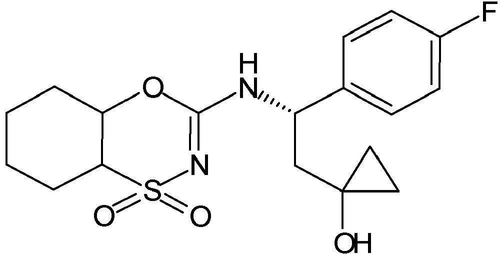 | I | 6,993 | 382,4 | 1-[(S)-2-(1,1-Dioxo-4a,5,6,7,8,8a-hexahydro-1H-1lambda6-benzo[1,4,3]oxathiazin-3-ylamino)-2-(4-fluoro-phenyl)-ethyl]-cyclopropanol * |
| 17 | 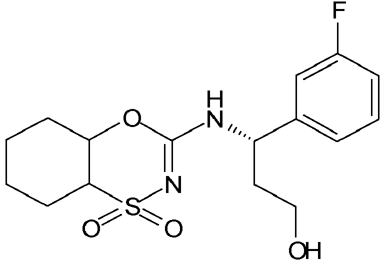 | A | 1,116 | 356,4 | (S)-3-(-1,1-Dioxo-4a,5,6,7,8,8a-hexahydro-1H-1lambda6-benzo[1,4,3]oxathiazin-3-ylamino)-3-(3-fluoro-phenyl)-propan-1-ol |
| 18 | 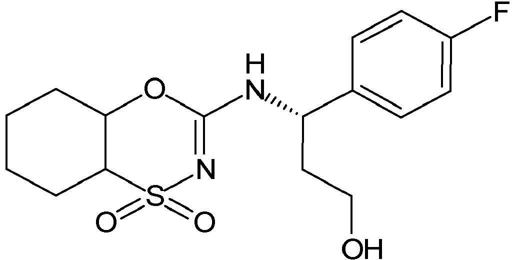 | I | 8,807 | 356,4 | (S)-3-(-1,1-Dioxo-4a,5,6,7,8,8a-hexahydro-1H-1lambda6-benzo[1,4,3]oxathiazin-3-ylamino)-3-(4-fluoro-phenyl)-propan-1-ol * |
| 19 | 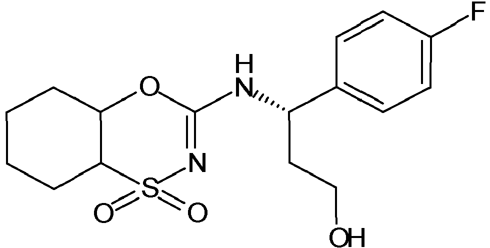 | I | 7,173 | 356,4 | (S)-3-(1,1-Dioxo-4a,5,6,7,8,8a-hexahydro-1H-1lambda6-benzo[1,4,3]oxathiazin-3-ylamino)-3-(4-fluoro-phenyl)-propan-1-ol * |
| 20 | 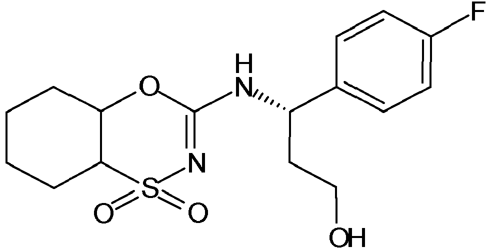 | I | 8,832 | 356,4 | (S)-3-(1,1-Dioxo-4a,5,6,7,8,8a-hexahydro-1H-1lambda6-benzo[1,4,3]oxathiazin-3-ylamino)-3-(4-fluoro-phenyl)-propan-1-ol * |
| 21 | 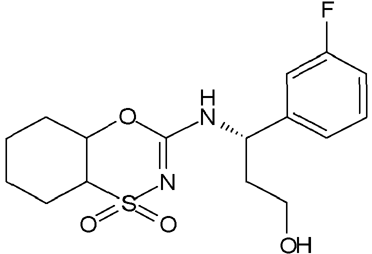 | I | 7,263 | 356,4 | (-)-(S)-3-(1,1-Dioxo-4a,5,6,7,8,8a-hexahydro-1H-1lambda6-benzo[1,4,3]oxathiazin-3-ylamino)-3-(3-fluoro-phenyl)-propan-1-ol * |
| 22 | 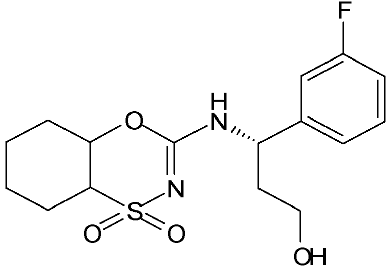 | I | 9,508 | 356,4 | (-)-(S)-3-(1,1-Dioxo-4a,5,6,7,8,8a-hexahydro-1H-1lambda6-benzo[1,4,3]oxathiazin-3-ylamino)-3-(3-fluoro-phenyl)-propan-1-ol * |
| 23 | 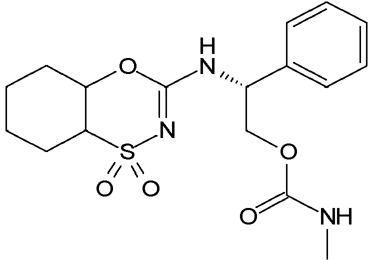 | B | 0,726 | 381,4 | Methyl-carbaminsäure (R)-2-(1,1-dioxo-4a,5,6,7,8,8a-hexahydro-1H-1lambda6-benzo[1,4,3]oxathiazin-3-ylamino)-2-phenyl-ethyl ester |
| 24 | 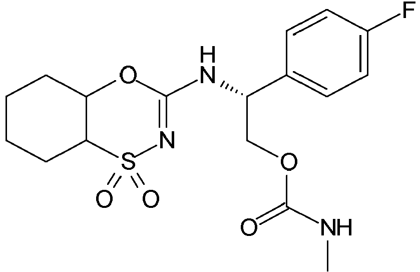 | B | 0,751 | 399,4 | Methyl-carbaminsäure (R)-2-(1,1-dioxo-4a,5,6,7,8,8a-hexahydro-1H-1lambda6-benzo[1,4,3]oxathiazin-3-ylamino)-2-(4-fluoro-phenyl)-ethyl ester |
| 25 | 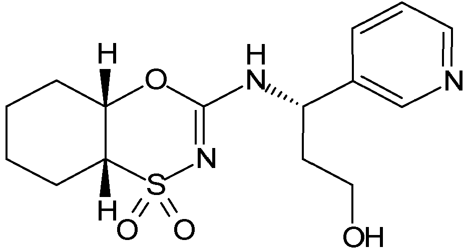 | B | 0,157 | 339,4 | (S)-3-((4aS,8aR)-1,1-Dioxo-4a,5,6,7,8,8a-hexahydro-1H-1lambda6-benzo[1,4,3]oxathiazin-3-ylamino)-3-pyridin-3-yl-propan-1-ol |
| 26 | 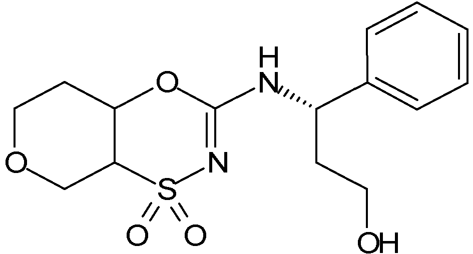 | B | 0,53 | 340,4 | (S)-3-(1,1-Dioxo-5,6,8,8a-tetrahydro-1H,4aH-4,7-dioxa-1lambda6-thia-2-aza-naphthalen-3-ylamino)-3-phenyl-propan-1-ol ** |
| 27 | 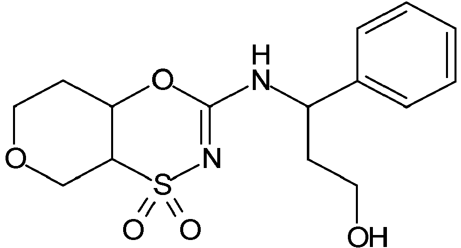 | B | 0,53 | 340,4 | (S)-3-(1,1-Dioxo-5,6,8,8a-tetrahydro-1H,4aH-4,7-dioxa-1lambda6-thia-2-aza-naphthalen-3-ylamino)-3-phenyl-propan-1-ol |
| 28 | 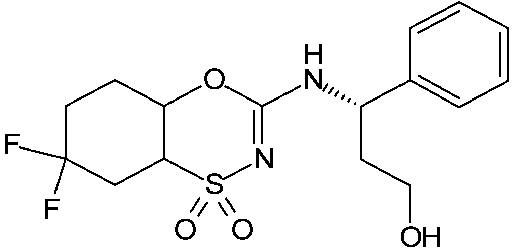 | B | 0,734 | 374,4 | (S)-3-(7,7-Difluoro-1,1-dioxo-4a,5,6,7,8,8a-hexahydro-1H-1lambda6-benzo[1,4,3]oxathiazin-3-ylamino)-3-phenyl-propan-1-ol |
| 29 | 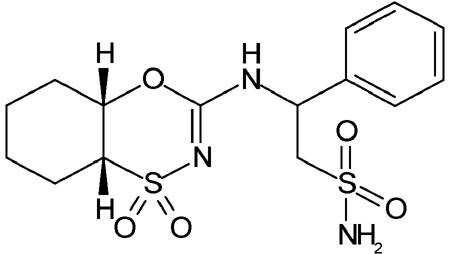 | B | 0,788 | 387,5 | 2-((4aS,8aR)-1,1-Dioxo-4a,5,6,7,8,8a-hexahydro-1H-1lambda6-benzo[1,4,3]oxathiazin-3-ylamino)-2-phenyl-ethanesulfonsäureamid * |
| 30 | 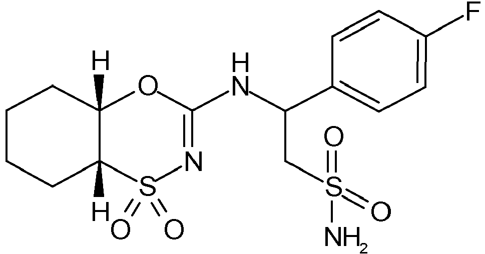 | B | 0,701 | 387,5 | 2-((4aS,8aR)-1,1-Dioxo-4a,5,6,7,8,8a-hexahydro-1H-1lambda6-benzo[1,4,3]oxathiazin-3-ylamino)-2-phenyl-ethanesulfonsäureamid * |
| 31 | 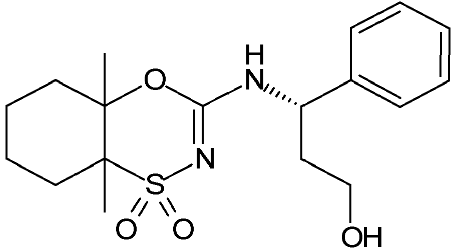 | C | 2,69 | 366,5 | (S)-3-(4a,8a-Dimethyl-1,1-dioxo-4a,5,6,7,8,8a-hexahydro-1H-1lambda6-benzo[1,4,3]oxathiazin-3-ylamino)-3-phenyl-propan-1-ol * |
| 32 | 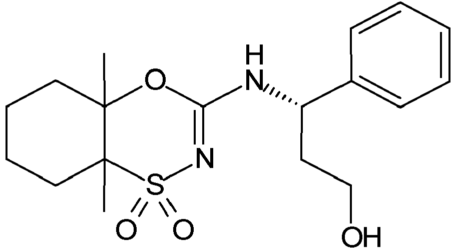 | C | 2,68 | 366,5 | (S)-3-(4a,8a-Dimethyl-1,1-dioxo-4a,5,6,7,8,8a-hexahydro-1H-1lambda6-benzo[1,4,3]oxathiazin-3-ylamino)-3-phenyl-propan-1-ol * |
| 33 | 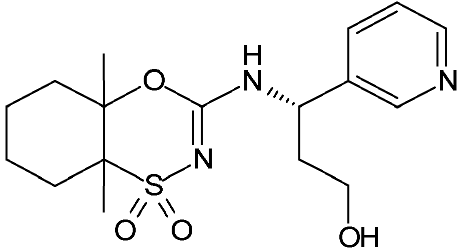 | C | 1,88 | 367,5 | (S)-3-(4a,8a-Dimethyl-1,1-dioxo-4a,5,6,7,8,8a-hexahydro-1H-1lambda6-benzo[1,4,3]oxathiazin-3-ylamino)-3-pyridin-3-yl-propan-1-ol * |
| 34 | 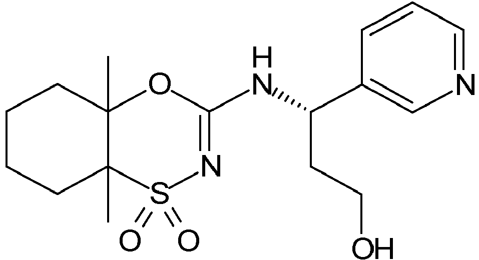 | C | 1,97 | 367,5 | (S)-3-(4a,8a-Dimethyl-1,1-dioxo-4a,5,6,7,8,8a-hexahydro-1H-1lambda6-benzo[1,4,3]oxathiazin-3-ylamino)-3-pyridin-3-yl-propan-1-ol * |

| | | | | | |
|---|---|---|---|---|---|
| * Isomerenreine Verbindung ** trans Verbindung | | | | | |

### Chromatographie-Methoden:

### Methode A

Säule: YMC J'spere ODS H80, 80 Ǻ, S-4 µm, 20 x 2.1 mm

Laufmittel: 0 min 90 % H₂O (0.05 % TFA) - 1.9 min 95 % Acetonitril - 2.4 min 95 % Acetonitril - 2.45 min 10 % Acetonitril (30 °C, Fluss 1 ml / min)

### Methode B

Säule: Mercury MS, Luna C18(2), S-3 µm, 10 x 2.0 mm

Laufmittel: 0 min 93 % H₂O (0.05 % TFA) - 1.2 min 95 % Acetonitril - 1.4 min 95 % Acetonitril - 1.45 min 7 % Acetonitril (30 °C, Fluss 1.1 ml / min)

### Methode C

Säule: Chiralpak AD-H/44, 5 µm, 250 x 4.6 mm

Laufmittel: Heptan : Ethanol : Methanol 5 : 1 : 1 (30 °C, Fluss 1 ml / min)

### Methode D

Säule: Chiralpak AD-H/39, 5 µm, 250 x 4.6 mm

Laufmittel: Heptan : Ethanol : Methanol 5 : 1 : 1 (30 °C, Fluss 1 ml / min)

### Methode E

Säule: Chiralpak AS-H/52, 5 µm, 250 x 4.6 mm

Laufmittel: Heptan : Ethanol : Methanol 5 : 1 : 1 (30 °C, Fluss 1 ml / min)

### Methode F

Säule: Chiralpak AS-H/52, 5 µm, 250 x 4.6 mm

Laufmittel: Heptan : Ethanol : Methanol 10 : 1 : 1 (30 °C, Fluss 1 ml / min)

### Methode G

Säule: YMC J'spere 33 x 2 mm 4 µM

Laufmittel: 0 min 95 % H₂O (0.05 % TFA) - 2.5 min 95 % Acetonitril (0.05 % TFA) - 3.0 min 95 % Acetonitril (30 °C, Fluss 1 ml / min)

### Methode H

Säule: Chiralpak AS-H/80, 5 µm, 250 x 4.6 mm

Laufmittel: Heptan : Ethanol : Methanol 2 : 1 : 1 (30 °C, Fluss 1 ml / min)

### Methode I

Säule: Chiralpak AS-H/80, 5 µm, 250 x 4.6 mm

Laufmittel: Heptan : Ethanol : Methanol 6 : 1 : 1 (30 °C, Fluss 1 ml / min)

### Methode J

Säule: YMC J'spere 33 x 2 mm 4 µM

Laufmittel: 0 min 98 % H₂O (0.05 % TFA) - 1.0 min 98 % Acetonitril (0.05 % TFA) - 5.0 min 95 % Acetonitril - 6.25 min 95 % Acetonitril (30 °C, Fluss 1 ml / min)

### Methode K

Säule: YMC J'spere 33 x 2 mm 4 µM

Laufmittel: 0 min 95 % H₂O (0.05 % TFA) - 0.5 min 95 % Acetonitril (0.05 % TFA) - 3.5 min 95 % Acetonitril - 4.0 min 95 % Acetonitril (30 °C, Fluss 1 ml / min)

### Methode L

Säule: YMC J'spere ODS H80, 80 Ǻ, S-4 µm, 20 x 2.1 mm

Laufmittel: 0 min 96 % H₂O (0.05 % TFA) - 2.0 min 95 % Acetonitril - 2.4 min 95 % Acetonitril - 2.45 min 4 % Acetonitril (30 °C, Fluss 1 ml / min)

### Methode M

Säule: Chiralpak AS-H/52, 5 µm, 250 x 4.6 mm

Laufmittel: Heptan : Ethanol : Methanol 10 : 1 : 1 , vorkonditioniert mit TFA (30 °C, Fluss 1 ml / min)

### Methode N

Säule: Chiralpak AD-H/44, 5 µm, 250 x 4.6 mm

Laufmittel: Heptan : Ethanol : Methanol 15 : 1 : 1 , vorkonditioniert mit TFA (30 °C, Fluss 1 ml / min)

### Methode C

Säule: Waters XBridge C18, 5 µm, 250 x 4.6 mm

Laufmittel: 0 min - 0.3 min 95 % H₂O (0.05 % TFA) + 5 % Acetonitril (0.05 % TFA) - 3.5 min 95 % Acetonitril (0.05 % TFA) - 4 min 95 % Acetonitril (0.05 % TFA) (30 °C, Fluss 1 ml / min)

Die Wirksamkeit der Verbindungen wurde wie folgt getestet:
Enzymatischer 11beta-HSD Test:

Zum Messen der Wirkung der Verbindungen wurde ein auf SPA basierendes Nachweisverfahren (Solly *et al.* 2005) angewendet. Zunächst wurden 20 µl der humanen 11β-HSD1-Mikrosomenfraktion (0,2 µg Protein), zubereitet in 50 mM HEPES, 0,1% BSA (w/v), in eine Platte mit 384 Vertiefungen gegeben. Die Testverbindungen (0,09 µl) wurden in 100% DMSO auf die Assayplatte übertragen. Die Reaktion wurde durch Zugabe von 20 µl [1,2-³H] Cortison (0,1 µCi/100 mM) in Assaypuffer mit 25 mM HEPES, 100 mM KCl, 5 mM NaCl, 2 mM MgCl₂ und 0,25 mM NADPH gestartet. Die Platte wurde 1 Stunde lang bei 37°C geschüttelt. Gleichzeitig wurde eine Stopplösung mit 20 mg/ml SPA-PVT-Perlen, 1,6 mg/ml monoklonalem Cortisol-Antikörper und 0,01 mM SSR110887 (Inhibitor aus dem Biovitrium-Patent) in 50 mM HEPES, 1 M NaCl und 1 M KCl bei Raumtemperatur gerührt. Zum Abbruch der Reaktion wurden in alle Vertiefungen jeweils 25 µl der Stopplösung gegeben. Die Platte wurde 1 weitere Stunde lang sachte bei Raumtemperatur geschüttelt und dann 1 min bei 500 gₐᵥ zentrifugiert, damit sich die SPA-Perlen absetzen konnten. Die Platte wurde dann in einem Wallac-1450-Microbeta-Gerät mit einem Standard-SPA-Programm gelesen (1 min Zählzeit/Vertiefung). Die Vergleichsverbindung war Glycyrrhetinsäure.
Protein und radioaktives Substrat wurden mit einem Biomek FX-Gerät (Beckman Coulter) zur Handhabung von Flüssigkeiten dispensiert. Die Testverbindungen wurden mit einem mit einem 90-nl-Pin-Tool ausgestatteten Cybi-Well (CyBio) zugesetzt.

Lit.: Solly S, Mundt SS, Zokian HJ, Juy-Fang Ding G, Hermanowski-Vosatka A, Strulovici B and Zheng W. High-throughput screening of 11β-Hydroxysteroid dehydrogenase type 1 in scintillation proximity format. Assay Drug Dev Technol 2005;3:377-384.

**Tabelle 2: Biologische Aktivität**

| Beispiel | IC50 (nM) |
|---|---|
| 1 | 31 |
| 2 | 575 |
| 3 | 24 |
| 4 | 63 |
| 5 | 8 |
| 6 | 4 |
| 9 | 5 |
| 10 | 104 |
| 12 | 3 |
| 13 | 5 |
| 14 | 236 |
| 17 | 27 |
| 18 | 4 |
| 19 | 11 |
| 20 | 13 |
| 25 | 25 |
| 26 | 742 |
| 27 | 62 |
| 28 | 445 |
| 29 | 15 |
| 30 | 11 |
| 31 | 34 |
| 32 | 4 |
| 33 | 9 |
| 34 | 353 |

Aus den Messdaten ist abzulesen, dass die Verbindungen der Formel I 11beta-HSD1 (11beta-Hydroxysteroid Dehydrogenase Type 1) inhibieren und dadurch gut zur Behandlung von Hyperglykämie, Insulin Resistenz, Diabetes, Adipositas, Lipidstoffwechselstörungen, Bluthochdruck, Verbesserung der Kognition, erhöhtem Augeninnendruck, der Förderung der Wundheilung und anderen Erkrankungen geeignet sind.

Nachfolgend wird die Herstellung einiger Beispiele detailliert beschrieben, die übrigen Verbindungen der Formel I wurden analog erhalten:
Experimenteller Teil:
   Die Verbindungen 1-22 konnten mit dieser Herstellungsvorschrift hergestellt werden:
      (S)-3-(1,1-Dioxo-4a,5,6,7,8,8a-hexahydro-1H-1lambda6-benzo[1,4,3]oxathiazin-3-ylamino)-3-phenyl-propan-1-ol

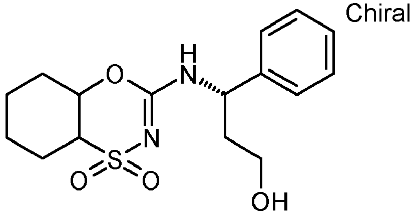

Unter Inertgas wurden 4 mmol (S)-3-Amino-3-phenyl-propan-1-ol und 8 mmol Diisopropylethyl-amin in 5 ml Dichlormethan vorgelegt und anschließend unter Eiskühlung 5 mmol 2-Chlorcyclohexansulfonylisocyanat in 5 ml Dichlormethan gelöst tropfenweise zugegeben und 1 Stunde gerührt. Die Vollständigkeit der Umsetzung wurde mit LCMS geprüft. Falls noch (S)-3-Amino-3-phenyl-propan-1-ol vorhanden war, wurden nochmals 0.4 eq 2-Chlorcyclohexansulfonylisocyanat zugegeben und über Nacht unter Rühren auf Raumtemperatur kommen gelassen. Die Reaktionslösung wurde mit 50 ml Dichlormethan verdünnt und 2 x mit 20 ml 10 %iger KHSO₄- Lösung extrahiert, die organische Phase über MgSO₄ getrocknet und einrotiert. Der Rückstand wurde dann mit 0.5 eq (bezogen auf das Amin) 0.1 N NaOH-Lösung versetzt. Zugabe von Dioxan (z.B. bei 20 ml 0.1M NaOH / 5ml Dioxan) und auf 100 °C erhitzt. Bei 100 °C entsteht eine klare Lösung. Falls noch etwas Ausgangsmaterial vorhanden ist, wurden 1-2 Tropfen 1 N NaOH zugegeben. Nach weiteren 30 Minuten bei 100 °C vollständige Umsetzung. Nach dem Abkühlen wurde mit 30 ml Dichlormethan versetzt und 2 x mit 10 ml gesättigter Na₂CO₃-Lösung gewaschen. Die wässrige Lösung wurde noch 1 x mit 20 ml Dichlormethan extrahiert. Die vereinigten organischen Phasen wurden über MgSO₄ getrocknet und einrotiert. Der Rückstand wurde in DMF gelöst und im Reinigungslabor über präparative HPLC gereinigt. Man erhielt so das Produkt (170 mg) mit dem Molekulargewicht 338.4 g / mol (C₁₆H₂₂N₂O₄S); MS (ESI): m / e = 339 (M+H+).

### 2-Chlor-cyclohexanesulfonyl isocyanat

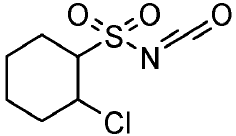

### Chemische Berichte 1970, 103(3), 663-669

Die verwendeten Amine sind käuflich oder können wie folgt hergestellt werden:

### (S)-4-Amino-4-phenyl-buttersäure-Natriumsalz

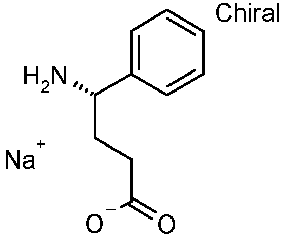

((S)-3-Cyano-1-phenyl-propyl)-carbaminsäure-tert-butyl ester (1.90 g) wurde in 6 N HCl (50 ml) suspendiert und für 16 Stunden bei 90 °C gerührt. Die Reaktionslösung wurde mit Dichlormethan gewaschen (50 ml), mit 12 N NaOH-Lösung basisch gestellt und eingeengt. Der Rückstand enthält das Produkt (1,5 g) mit dem Molekulargewicht 179.2 g / mol (C₁₀H₁₃NO₂); MS (ESI): m / e = 180 (M+H+).

### (S)-4-Amino-4-phenyl-butan-1-ol

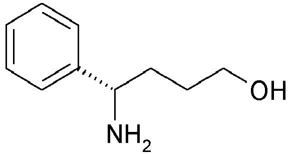

(S)-4-Amino-4-phenyl-buttersäure-Natriumsalz (1.48 g) wurde portionsweise zu einer Suspension von Lithiumaluminiumhydrid (1.00 g) in THF (100 ml) gegeben. Die Mischung wurde für 4 Stunden bei 50 °C gerührt.und anschließend nacheinander mit Wasser (1 ml), 6 N NaOH-Lösung (3 ml) und wieder mit Wasser (3 ml) versetzt und filtriert. Das Filtrat wurde eingeengt und der Rückstand säulenchromatographisch gereinigt. Man erhielt so das Produkt (70 mg) mit dem Molekulargewicht 165.2 g / mol (C₁₀H₁₅NO); MS (ESI): m / e = 166 (M+H+).

### (R)-4-Amino-4-phenyl-butan-1-ol wurde analog hergestellt:

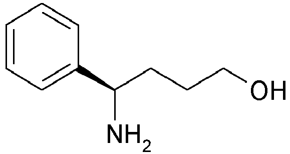

### ((S)-3-Cyano-1-phenyl-propyl)-carbaminsäure-tert-butyl ester

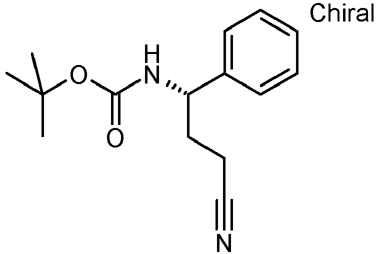

Eine Lösung von Methansulfonsäure-(S)-3-tert-butoxycarbonylamino-3-phenyl-propylester (2.0 g) und Natriumcyanid (0.97 g) in DMF (10 ml)wurde bei 70 °C für 16 Stunden gerührt. Die Reaktionsmischung wurde zwischen Wasser (50 ml) und Essigester (50 ml) verteilt und die wässrige Phase wurde reextrahiert (2 x 50 ml). Die vereinigten organischen Phasen wurden über Magnesiumsulfat getrocknet und eingeengt. Man erhielt so das Produkt (1,7 g) mit dem Molekulargewicht 260.3 g / mol (C₁₅H₂₀N₂O₂); MS (ESI): m / e = 261 (M+H+).

### Methansulfonsäure-(S)-3-tert-butoxycarbonylamino-3-phenyl-propylester

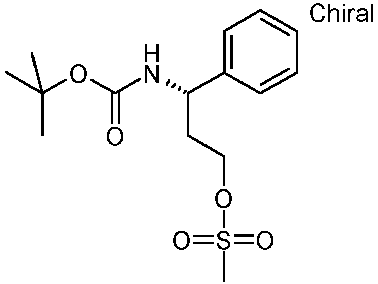

Zu einer Mischung aus (S)-3-Amino-3-phenyl-propan-1-ol Hydrochlorid (1.50 g) und Triethylamin (2.80 ml) in Dichlormethan (100 ml) wurde BOC-anhydrid (1.92 g) gegeben und die Mischung wurde wurde für 1 Stunde bei Raumtemperatur gerührt. Die Lösung wurde mit Wasser und Kaliumcarbonatlösung gewaschen und über Magnesiumsulfat getrocknet.Zur resultierenden Lösung wurde Pyridin (0.97 ml) gegeben und bei 0 °C tropfenweise Methansulfonsäurechlorid (0.68 ml). Die Lösung wurde bei Raumtemperatur für 5 Stunden gerührt und anschließend mit 1 N HCl und Wasser gewaschen, über Magnesiumsulfat getrocknet und eingeengt. Man erhielt so das Produkt (2,0 g) mit dem Molekulargewicht 329.4 g / mol (C₁₅H₂₃NO₅S); MS (ESI): m / e = 330 (M+H+).

### Carbaminsäure-(R)-2-amino-2-phenylethylester

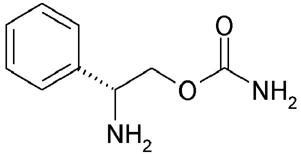

(R)-2-Amino-2-phenyl-ethanol (0.60 g) wurde mit BOC-anhydrid (1,42 g) in Dichlormethan (10 ml) bei Raumtemperatur für 8 Stunden gerührt. Die Reaktionslösung wurde mit Natriumcarbonatlösung gewaschen, über Magnesiumsulfat getrocknet und eingeengt. Der Rückstand wurde in Dichlormethan (30 ml) gelöst und bei 0 °C mit Trichloracetylisocyanat (1.02 g) versetzt. Die Mischung wurde 2 Stunden bei Raumtemperatur gerührt und eingeengt. Der Rückstand wurde in Methanol (15 ml) aufgenommen, mit Kaliumcarbonatlösung (20 ml) versetzt und 5 Stunden bei Raumtemperatur gerührt. Das ausgefallene Produkt wurde abgesaugt, mehrfach mit Wasser gewaschen und getrocknet. Das Produkt wurde in Dichlormethan (10 ml) gelöst, mit Trifluoressigsäure (5 ml) versetzt und für 1.5 Stunden bei Raumtemperatur gerührt. Das Lösungsmittel wurde im Vakuum entfernt und der Rückstand wurde in Dichlormethan aufgenommen, mit verdünnter Natronlauge gewaschen, über Magnesiumsulfat getrocknet und eingeengt. Man erhielt so das Produkt (533 mg) mit dem Molekulargewicht 180.2 g / mol (C₉H₁₂N₂O₂); MS (ESI): m / e = 181 (M+H+). Carbaminsäure-(S)-2-amino-2-phenylethylester wurde analog hergestellt:

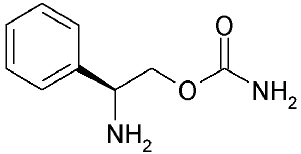

### 1-[(S)-2-Amino-2-(4-fluor-phenyl)-ethyl]-cyclopropanol

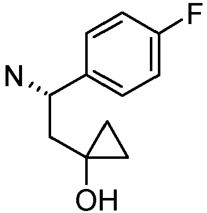

(S)-Methyl 3-amino-3-(4-fluorphenyl)-propionsäureester Hydrochlorid (4.5 g) wurden in THF (30 ml) gelöst und auf 0 °C abgekühlt. Nach der Zugabe von Diisopropyl-ethylamin (8.8 g) wurde langsam Chlorameisensäurebenzylester (4.3 g) zugetropft und 1 Stunde bei 0 °C gerührt. Das Reaktionsgemisch wurde einrotiert, mit Dichlormethan (50 ml) versetzt und mit gesättigter NH₄Cl-Lösung 2 x gewaschen. Anschließend wurde die organische Phase mit MgSO₄ getrocknet, filtriert und konzentriert. Der Rückstand (5.72 g) wurde in THF aufgenommen, mit Titantetraisopropoxid (0.49 g) versetzt und über 1 Stunde Ethylmagnesiumbromid (3 N, 17.9 ml) zugetropft. Nach 24 Stunden wurde die Reaktionslösung in kalte 2 N Schwefelsäure (150 ml) gegossen und mit Dichlormethan 3 x extrahiert. Die vereinten organischen Phasen wurden mit MgSO₄ getrocknet, filtriert und konzentriert. Der Rückstand wurde mit präparativer HPLC gereinigt. Der erhaltene (S)-1-(4-Fluor-phenyl)-2-(1-hydroxy-cyclopropyl)-ethyl]-carbaminsäurebenzylester (0.76 g) wurde in Methanol (20 ml) gelöst, mit Pd/C 5 % versetzt und unter 2 bar Druck Wasserstoff hydriert. Nach Filtration und Konzentration erhielt man so das Produkt (87 mg) mit dem Molekulargewicht 329.3 g / mol (C₁₉H₂₀FNO₃), MS (ESI): m / e = 330 (M+H+).

### ((R)-2-Hydroxy-1-phenyl-ethyl)carbaminsäure-tert-butylester

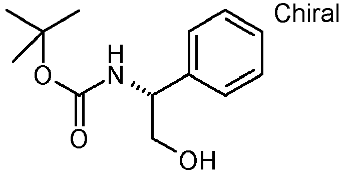

(R)-2-Amino-2-phenyl-ethanol (3 g) wuden in Dichlormethan gelöst und bei Raumtemperatur mit BOC-anhydrid (4.8 g) tropfenweise versetzt. Die Mixtur wurde anschließend 2.5 Stunden bei Raumtemperatur gerührt. Das Lösungsmittel wurde im Vakuum entfernt. Die Substanz wurde ohne weitere Aufarbeitung weiterverwendet.

### (S)-3-(tert-Butyl-dimethyl-silanyloxy)-1-phenyl-propylamin

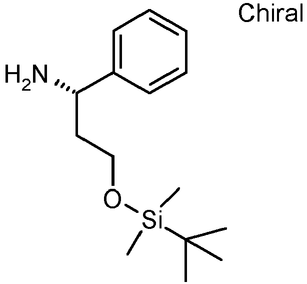

S-3-Amino-3-phenyl-1-propanol (4.72 g) wurde in Dichlormethan (60 ml) gelöst, mit Triethylamin (6.36 g) und tert-Butyl-dimethyl-chlorsilan (4.1 g) versetzt und 3 Stunden bei Raumtemperatur gerührt. Dann wurde mit Wasser (3 x 50 ml) gewaschen und über eine Phasenseparatorkartusche getrocknet. Man erhielt so das Produkt (7 g) mit dem Molekulargewicht 265.5 g / mol (C₁₅H₂₇NOSi), MS (ESI): m / e = 266 (M+H+).

### Methyl-carbaminsäure (R)-2-phenyl-ethyl ester

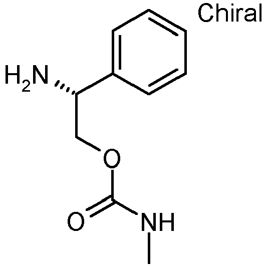

((R)-2-Hydroxy-1-phenyl-ethyl)carbaminsäure-tert-butylester (1.2 g), Carbonyldiimidazol (930 mg) und Methylamin (2 N Lösung in THF, 3.8 ml), wurden in THF (10 ml) gelöst und 16 Stunden bei Raumtemperatur gerührt. Dann wurde Ethylacetat (50 ml) zugegeben und die organische Lösung mit Kaliumhydrogensulfat-Lösung (2 x 20 ml), gesättigter Natriumhydrogencarbonat-Lösung (1 x 20 ml) und mit gesättigter Natriumchlorid-Lösung (1 x 20 ml) gewaschen, über eine Kieselgurkartusche getrocknet und im Vakuum konzentriert. Der Rückstand wurde anschließend mit Ethylacetat / n-Heptan mit Normalphasenchromatographie gereinigt. Nach dem Vereinigen der Produktfraktionen wurde im Vakuum konzentriert, der Rückstand (550 mg) mit Dichlormethan (3 ml) und Trifluoressigsäure (1 ml) versetzt, und 1 Stunde bei Raumtemperatur gerührt. Nach Zugabe von Dichlormethan (20 ml) wurde mit Natriumhydrogencarbonat-Lösung (2 x 20 ml) und gesättigter Natriumchlorid-Lösung (1 x 20 ml) gewaschen, getrocknet (Natriumsulfat), filtriert und im Vakuum konzentriert. Man erhielt so das Produkt (259 mg) mit dem Molekulargewicht 194.3 g / mol (C₁₀H₁₄N₂O₂), MS (ESI): m / e = 195 (M+H+).

Auf die gleiche Weise wurde folgende Verbindung hergestellt:
Methyl-carbaminsäure (R)-2-amino-2-(4-fluor-phenyl)-ethylester

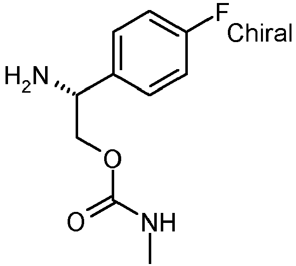

Die Verbindungen 23, 24, 29, 30 konnten mit dieser Herstellungsvorschrift hergestellt werden:

### (S)-3-((4aS,8aR)-1,1-Dioxo-4a,5,6,7,8,8a-hexahydro-1H-1lambda6-benzo[1,4,3]oxathiazin-3-ylamino)-3-pyridin-3-yl-propionsäure ethylester

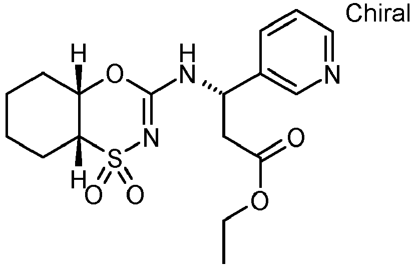

(S)-3-(4aS,8aR)-Amino-3-pyridin-3-yl-propionsäureethylester Dihydrochlorid (1 g) wurde in trockenem Dichlormethan (10 ml) mit Triethylamin (2.05 m) versetzt und 5 Minuten bei Raumtemperatur gerührt. 1,1'Thiocarbonyldiimidazol (717 mg) wurde zugesetzt und dann weitere 30 Minuten gerührt. Die Lösung wurde mit Diethylether / n-Pentan 1 : 1 (20 ml) verdünnt, mit Wasser (20 ml) gewaschen, mit Natriumsulfat getrocknet, filtriert und im Vakuum konzentriert. Der Rückstand wurde in NMP (4 ml) aufgenommen (Lösung 1). Cis-2-Hydroxy-cyclohexanesulfonsäureamid (488 mg), gelöst in NMP (6 ml), wurde auf 0 °C abgekühlt und mit Natrium-bis(trimethylsilyl)amid (2 N Lösung, 1.36 ml) versetzt und 5 Minuten gerührt. Lösung 1 wurde dann bei 0 °C zugesetzt und weitere 5 Minuten gerührt. Anschließend wurde N-Bromsuccinimid (485 mg) zugesetzt und für 3 Minuten gerührt. Es wurde dann mit Wasser (100 ml) verdünnt und mit Ethylacetat (3 x 30 ml) extrahiert. Die vereinigten organischen Phasen wurden mit Natriumsulfat getrocknet, filtriert und im Vakuum konzentriert. Die Reinigung erfolgte über präparative HPLC. Man erhielt so das Produkt (214 mg) mit dem Molekulargewicht 381.4 g / mol (C₁₇H₂₃N₃O₅S), MS (ESI): m / e = 382 (M+H+).

### (S)-3-((4aS,8aR)-1,1-Dioxo-4a,5,6,7,8,8a-hexahydro-1H-1lambda6-benzo[1,4,3]oxathiazin-3-ylamino)-3-pyridin-3-yl-propan-1-ol

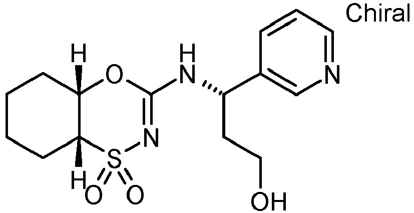

(S)-3-(1,1-Dioxo-4a,5,6,7,8,8a-hexahydro-1H-1lambda6-benzo[1,4,3]oxathiazin-3-ylamino)-3-pyridin-3-yl-propionsäureethylester (200 mg) wurde in trockenem Dichlormethan (15 ml) gelöst und bei -70 °C mit Diisobutylaluminiumhydrid (1 N Lösung, 2.65 ml) versetzt und 1 Stunde gerührt. Das Gemisch wurde mit Aceton (0.25 ml) und Essigsäure (0.3 ml) versetzt und auf Raumtemperatur gebracht. Das Lösungsmittel wurde im Vakuum entfernt, mit Methanol (15 ml) verdünnt und Natriumborhydrid (198 mg) in kleinen Portionen bei Raumtemperatur zugesetzt. Nach 1 Stunde wurde gesättigte, wässrige Rochelles Salzlösung zugegeben und 1 weitere Stunde gerührt. Es wurde filtriert und das Filtrat im Vakuum konzentriert. Die Reinigung erfolgte über präparative HPLC. Man erhielt so das Produkt (23 mg) mit dem Molekulargewicht 339.4 g / mol (C₁₅H₂₁N₃O₄S), MS (ESI): m / e = 340 (M+H+) als Hydrochlorid-Salz.

Die Verbindungen 26-28 konnten mit dieser Herstellungsvorschrift hergestellt werden:

### (S)-3-(1,1-Dioxo-5,6,8,8a-tetrahydro-1H,4aH-4,7-dioxa-1lambda6-thia-2-aza-naphthalen-3-ylamino)-3-phenyl-propan-1-ol

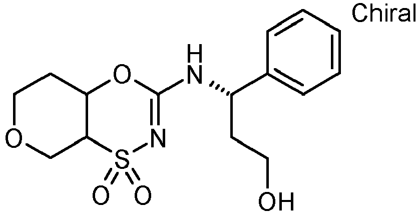

(S)-3-(tert-Butyl-dimethyl-silanyloxy)-1-phenyl-propylamin (273 mg) wurde in trockenem Dichlormethan (2 ml) gelöst. 1,1'Thiocarbonyldiimidazol (216 mg) wurde zugesetzt und dann weitere 45 Minuten gerührt. Die Lösung wurde mit Diethylether / n-Pentan 1 : 1 (20 ml) verdünnt, mit Wasser (20 ml) gewaschen, mit Natriumsulfat getrocknet, filtriert und im Vakuum konzentriert. Der Rückstand wurde in NMP (1 ml) aufgenommen (Lösung 1). 4-Hydroxy-tetrahydro-pyran-3-sulfonsäureamid (200 mg), gelöst in NMP (2 ml), wurde auf 0 °C abgekühlt und mit Natrium-bis(trimethylsilyl)amid (2 N Lösung, 0.55 ml) versetzt und 5 Minuten gerührt. Lösung 1 wurde dann bei 0 °C zugesetzt und weitere 10 Minuten gerührt. Anschließend wurde N-Bromsuccinimid (196 mg) zugesetzt und für 5 Minuten gerührt. Es wurde dann mit Wasser (100 ml) verdünnt und mit Ethylacetat (3 x 30 ml) extrahiert. Die vereinigten organischen Phasen wurden mit Natriumsulfat getrocknet, filtriert und im Vakuum konzentriert. Der Rückstand wurde in Methanol (3 ml) aufgenommen, mit konzentrierter HCl (0.3 ml) versetzt und 90 Minuten bei Raumtemperatur gerührt. Dann wurde mit Natriumhydroxid-Lösung 1 N neutralisiert und im Vakuum konzentriert. Die Reinigung erfolgte über präparative HPLC. Man erhielt so die cis / trans Produkte (33 mg, 19 mg) mit dem Molekulargewicht 340.4 g / mol (C₁₅H₂₀N₂O₅S), MS (ESI): m / e = 341 (M+H+).

### Sulfamoylchlorid

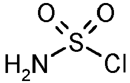

Zu einer Lösung von Chlorsulfonylisocyanat (32.5 g) in Methylenchlorid (50 ml) wurde über 10 Minuten bei 0 °C Ameisensäure in Methylenchlorid (50 ml) portionsweise zugegeben. Nach 30 Minuten bei Raumtemperatur wurde nochmals Methylenchlorid (50 ml) zugegeben. Nach 16 Stunden war eine klare, leicht gelblich schimmernde Flüssigkeit entstanden. Danach wurde der Ansatz 1 Stunde im Tiefkühlschrank auf -18 °C abgekühlt. Der entstandene Niederschlag wurde abgesaugt und im Vakuumtrockenschrank getrocknet.Der getrocknete Niederschlag (15.8 g) wurde unter Argon bei -18 °C gelagert.

### 2-Oxo-cyclohexanesulfonsäureamid

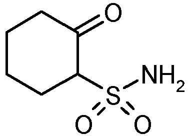

1-Piperidino-cyclohexen (11.3 g) wurde in THF (100 ml), wasserfrei, unter Argon gelöst. Danach wurde der Ansatz auf - 60 °C abgekühlt und Sulfamoylchlorid (11.85 g) in THF (100 ml), wasserfrei, wurde tropfenweise zugegeben (Temperaturerhöhung von -60 °C auf etwa -40 °C). Der Ansatz wurde wieder auf -60 °C abgekühlt. Nun wurde eine Lösung aus Hünig-Base (13.3 g) in THF (50 ml), wasserfrei, langsam zugegeben. Der Ansatz wurde auf Raumtemperatur erwärmt und 2 Stunden gerührt. Ein nicht rührbares Öl entstand. Das THF wurde abdekantiert, der ölige Rückstand in Methanol gelöst und über Kieselgel filtriert. Anschließend wurde das Methanol im Vakuum entfernt. Der Rückstand wurde in Essigester / n-Heptan 1 : 1 (40 ml) in der Siedehitze vollständig gelöst. Nach dem Abkühlen auf Raumtemperatur wurde im Kühlschrank auf 5 °C abgekühlt. Das Präzipitat wurde anschließend über eine Fritte abgesaugt. Man erhielt so das Produkt (5.3 g) mit dem Molekulargewicht 177.2 g / mol (C₆H₁₁NO₃S), MS (ESI): m / e = 178 (M+H+).

Auf die gleiche Weise wurden folgende Verbindungen hergestellt:
4-Oxo-tetrahydro-pyran-3-sulfonsäureamid 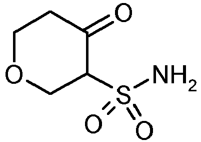 5,5-Difluor-2-oxo-cyclohexanesulfonsäureamid 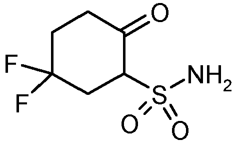
N-Sulfinyl-p-toluamid

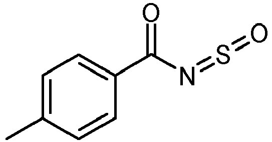

p-Toluamid (76 g) und Pyridin (95.5 ml) wurden in trockenem Ether (600 ml) vorgelegt und unter Eiskühlung Thionylchlorid (70.2 g) in Ether (200 ml) zugetropft. Es wurde über Nacht bei Raumtemperatur gerührt. Unter Feuchtigkeitsausschluss wurde dann das Pyridinhydrochlorid abfiltriert und mit Ether (100 ml) nachgewaschen. Die Lösung wurde im Vakuum konzentriert und der Rückstand (83.5 g) ohne weitere Reinigung verwendet.

### 3-p-Tolyl-4a,5,6,7,8,8a-hexahydro-benzo[1,4,3]oxathiazin 1,1-dioxid

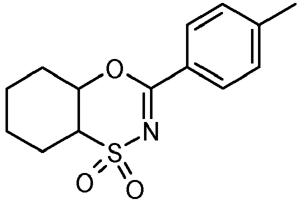

N-Sulfinyl-p-toluamid (83.3 g) wurden in Dichlormethan (300 ml) gelöst und mit Cyclohexen (81.1 g) versetzt und 24 Stunden gerührt. Die Reaktionslösung wurde einrotiert, in Ether (100 ml) aufgenommen und über eine Kieselgelsäule (350 g) wie folgt das Produkt isoliert. 1.5 1 Dichlormethan / Ether 1:1; 500 ml Dichlormethan / Ether 3:2; 500 ml Dichlormethan / Ether 4:1; jeweils 200 ml-Fraktionen. Die produkthaltigen Fraktionen wurden vereinigt, einrotiert und der Rückstand mit 150 ml Ether/ 100 ml n-Pentan verrührt. Ausgefallenes Produkt wurde abfiltriert, mit n-Pentan (50 ml) nachgewaschen und getrocknet. Anschließend wurde im Vakuum konzentriert. Der Rückstand (24.6 g) wurde in Dichlormethan (200 ml) aufgenommen und portionsweise mit 3-Chlorperoxybenzoesäure versetzt, sodass die Temperatur 30 °C nicht überschritten wurde. Nach beendeter Zugabe (45 Minuten) wurde über Nacht nachgerührt. Die ausgefallene 3-Chlorbenzoesäure wurde abgesaugt, mit Dichlormethan (2 x 500 ml), Natriumhydroxid (0.5 N Lösung, 100 ml) und mit Natriumchlorid-Lösung (halbkonzentriert, 250 ml) gewaschen, über MgSO₄ getrocknet, filtriert und im Vakuum einrotiert. Der kristalline Rückstand (11.9 g) wurde mit Ether/n-Pentan 1:1 (100 ml) aufgeschlämmt, abgesaugt, mit n-Pentan (100 ml) gewaschen und im Hochvakuum getrocknet. Das Produkt wurde in seine Enantiomere getrennt:
Säule: Chiralpak AS-H/75, 5 µm, 250 x 4.6 mm
Laufmittel: Heptan : iso-Propanol : Methanol 3 : 2 : 2 (30 °C, Fluss 1 ml / min)
Retentionszeiten: 7.728 Minuten und 9.257 Minuten.

### 4-Hydroxy-tetrahydro-pyran-3-sulfonsäureamid

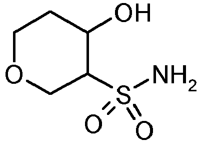

4-Oxo-tetrahydro-pyran-3-sulfonsäureamid (1.09 g) wurde in THF / Methanol 2 : 1 (110 ml) gelöst, Natriumborhydrid (0.23 g) portionsweise zugesetzt und 18 Stunden bei Raumtemperatur gerührt. Mit konzentrierter Salzsäure wurde dann auf pH 2 eingestellt und dann das Lösungsmittel im Vakuum entfernt. Der Rückstand wurde chromatographisch gereinigt (Lösungsmittel Dichlormethan / Methanol). Man erhielt so das Produkt (0.65 g) mit dem Molekulargewicht 181.2 g / mol (C₅H₁₁NO₄S).

Auf die gleiche Weise wurde folgende Verbindung hergestellt:
5,5-Difluor-2-hydroxy-cyclohexansulfonsäureamid 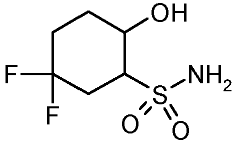
2-Hydroxy-cyclohexansulfonsäureamid

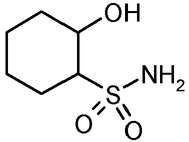

3-p-Tolyl-4a,5,6,7,8,8a-hexahydro-benzo[1,4,3]oxathiazin 1,1-dioxid (9.94 g) wurde in halbkonzentrierter wässriger Natriumhydroxid-Lösung (50 ml) und Ethanol (10 ml) als Lösevermittler auf 100 °C für 18 Stunden erhitzt. Dann wurde der Ethanol am Rotationsverdampfer entfernt, die wässrige Lösung mit konzentrierter HCl auf pH 3.8 eingestellt, auf Raumtemperatur abgekühlt, die ausgefallene Tolylsäure abgesaugt, mit wenig Wasser (10 ml) nachgewaschen und die wässrige Lösung mit Dichlormethan (100 ml) gewaschen. Anschließend wurde mit n-Butanol (5 x 50 m) das Produkt aus der wässrigen Phase extrahiert, das Lösungsmittel am Rotationsverdampfer entfernt, der Rückstand in Ethanol (100 ml) gelöst und mit Dichlormethan (100 ml) versetzt. Etwas ungelöster Feststoff wurde abfiltriert und die Lösung einrotiert. Anschließend wurde der Rückstand in Wasser (100 ml) gelöst und gefriergetrocknet. Man erhielt so das Produkt (3.24 g) mit dem Molekulargewicht 179.2 g / mol (C₆H₁₃NO₃S), MS (ESI): me / e = 180 (M+H+).

### 2-Amino-2-phenyl-ethansulfonsäureamid

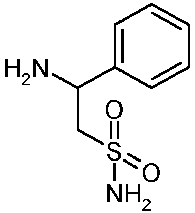

Zu einer heißen Lösung aus Hydroxylamin-hydrochlorid (27 mg) in Wasser (0.02 mL) wurde in Ethanol (0.5 mL) gelöstes Natriumethoxylat (130 mg) zugegeben und anschließend die Lösung auf 5 °C abgekühlt. Die Suspension wurde filtriert und das Filtrat mit 2-Phenyleth-1-en-1-sulfonamid (35 mg) versetzt. Dann wurde bei 100 °C 7 Stunden gerührt und im Anschluss im Vakuum konzentriert. Man erhielt so das Produkt mit dem Molekulargewicht 216.3 g / mol (C₈H₁₂N₂O₃S), MS (ESI): m / e = 217 (M+H+).

Die Substanz (1.81 g aus größerem Ansatz) wurde ohne weitere Aufarbeitung in 1 N HCl / AcOH 2 : 1 gelöst, dazu Zink gegeben und bei Raumtemperatur gerührt. Anfangs wurden 10 Equivalente Zink zugegeben, nach 16 Stunden wurden weiter 18 Equivalente Zink zugegeben und weitere 2 Stunden gerührt. Die Reaktionslösung wurde mit konzentrierter Natriumhydoxid-Lösung auf pH 9 eingestellt, der entstandene Niederschlag über Celite abfiltriert und dann mit Methanol (3 x 5 ml) nachgewaschen. Zur Reinigung der Substanz wurde 2 Tage mit BOC-anhydrid (1.2 Equivalente) gerührt und anschließend das Methanol im Vakuum entfernt. Nach Zugabe von Wasser (30 ml) wurde mit Ethylacetat (2 x 30 ml) extrahiert, über MgSO₄ getrocknet und im Vakuum einrotiert. Der Rückstand wurde über Normalphase gereinigt (20 g Kieselgelsäule; Produkt eluiert bei ca. 50 % Ethylacetat-Anteil). Die Wertfraktionen wurden vereinigt und einrotiert. Der Rückstand wurde in Dioxan (20 ml) aufgenommen, mit Salzsäure (4 N, 10 ml) versetzt und 2 Stunden bei Raumtemperatur gerührt. Anschließend wurde im Vakuum konzentriert, noch 2 x Methanol zugesetzt und wieder konzentriert, um Wasserreste zu entfernen, und dann im Hochvakuum getrocknet. Man erhielt so das Produkt (312 mg) mit dem Molekulargewicht 200.3 g / mol (C₈H₁₂N₂O₂S), MS (ESI): m / e = 201 (M+H+) als Hydrochlorid-Salz.

### N-tert-Butyl-methansulfonamid

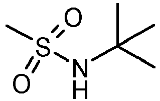

Zu einer Lösung aus Methansulfonylchlorid (7.7 ml) in Dichlormethan (100 ml) wurde tropfenweise tert-Butylamin (23.1 ml) bei 0 °C zugegeben und 5 Minuten gerührt. Anschließend wurde filtriert und das Filtrat mit 1 N Salzsäure (100 ml) gewaschen, mit Magnesiumsulfat getrocknet, filtriert und im Vakuum konzentriert. Man erhielt so das Produkt (14.7 g) mit dem Molekulargewicht 151.3 g/mol (C₅H₁₃NO₂S).

### 2-(4-Fluor-phenyl)-2-oxo-ethansulfonsäure tert-butylamid

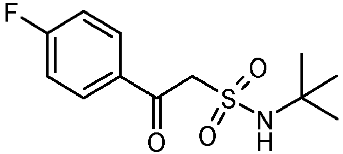

N-tert-Butyl-methansulfonamid (1.00 g) wurde in THF (10 ml) vorgelegt und auf -78 °C gekühlt. n-Buthyllithium (1.6 N in Hexan, 9.00 ml) wurde tropfenweise zugegeben. Anschließend wurde innerhalb von 5 Minuten auf 0°C erwärmt, 5 weitere Minuten gerührt und wiederum auf -78 °C gekühlt. 4-Fluorbenzoesäuremethylester (1.56 g) in THF (10 ml) gelöst und auf -78 °C gekühlt. Die oben hergestellte Lösung wurde tropfenweise bei tiefer Temperatur zugegeben. Nach beendeter Zugabe wurde auf Raumtemperatur kommen gelassen und 1 Stunde nachgerührt. Nach der Zugabe von Essigsäure (0.82 ml) und Ethylacetat (50 ml) wurde mit gesättigter Natriumhydrogencarbonat-Lösung (50 ml) und gesättigter Natriumchlorid-Lösung (50 ml) gewaschen. Die organische Phase wurde über Magnesiumsulfat getrocknet, filtriert und einrotiert. Die Reinigung erfolgte über Normalphase mit einem n-Heptan /Ethylacetat-Gradienten. Die Wertfraktionen wurden vereinigt, einrotiert und im Vakuum getrocknet. Man erhielt so das Produkt (661 mg) mit dem Molekulargewicht 273.3 g / mol (C₁₂H₁₆FNO₃S), MS (ESI): m / e = 296 (M+Na+).

### 2-Amino-2-(4-fluor-phenyl)-ethansulfonsäure tert-butylamid

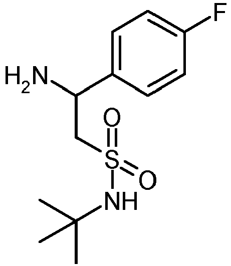

Eine Lösung aus 2-(4-Fluor-phenyl)-2-oxo-ethansulfonsäure tert-butylamid (657 mg), Ammoniumacetat (1.85 g) und Natriumcyanborhydrid (166 mg) wurden in Methanol (12 ml) 17 Stunden bei 60 °C gerührt. Die Reaktionslösung wurde einrotiert und der Rückstand in Ethylacetat (50 ml) aufgenommen. Dann wurde mit 0.5 N wässriger Natriumhydroxid-Lösung (50 ml) und mit gesättigter Natriumchlorid-Lösung (50 ml) gewaschen, die organische Phase über MgSO₄ getrocknet, filtriert und im Vakuum einrotiert. Man erhielt so das Produkt (697 mg) mit dem Molekulargewicht 274.3 g / mol (C₁₂H₁₉FN₂O₂S), MS (ESI): m / e = 275 (M+H+).

### 2-((4aS,8aR)-1,1-Dioxo-4a,5,6,7,8,8a-hexahydro-1H-1lambda6-benzo[1,4,3]oxathiazin-3-ylamino)-2-(4-fluor-phenyl)-ethansulfonsäureamid

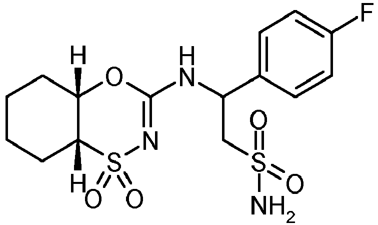

2-((4aS,8aR)-1,1-Dioxo-4a,5,6,7,8,8a-hexahydro-1H-1lambda6-benzo[1,4,3]oxathiazin-3-ylamino)-2-(4-fluor-phenyl)-ethansulfonsäure tert-butylamid (50 mg) wurde mit Trifluoressigsäure (1 ml) versetzt und 5 Stunden bei Raumtemperatur gerührt. Die Lösung wurde dann mit präparativer HPLC gereinigt. Man erhielt so das Produkt (697 mg) mit dem Molekulargewicht 405.4 g / mol (C₁₅H₂₀FN₃O₅S₂), MS (ESI): m / e = 406 (M+H+). 4a,8a-Dimethyl-3-(4-nitro-phenoxy)-4a,5,6,7,8,8a-hexahydro-benzo[1,4,3]oxathiazin 1,1-dioxid

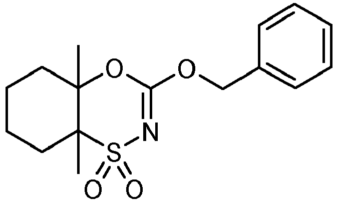

Zu einer Lösung aus Chlorsulfonylisocyanat (6 g) in Dichlormethan (75 ml) wurde Benzylalkohol (4.58 g) gegeben und die Lösung bei Raumtemperatur 1 Stunde gerührt. Anschließend wurde das Lösungsmittel im Vakuum entfernt. Der Rückstand wurde in THF (50 ml) aufgenommen und mit Natriumhydrid (95 %, 1.77 g) bei -78 °C versetzt. Die Kühlung wurde entfernt und der Ansatz langsam auf Raumtemperatur erwärmt. Dann wurde 1,2-Dimethyl-cyclohexen (4.17 g) zugegeben und auf 35 °C erwärmt. Nach 3 Stunden wurde auf Eis (100 g) gegossen und anschließend mit Ethylacetat (2 x 100 ml) extrahiert. Die organische Phase wurde mit Natriumsulfat getrocknet, filtriert und im Vakuum konzentriert. Der Rückstand (8 g) wurden mit präparativer HPLC gereinigt. Man erhielt so das Produkt (2.65 g) mit dem Molekulargewicht 323.4 g / mol (C₁₅H₂₁NO₄S). Die Trennung in die reinen Enantiomere erfolgte mit chiraler HPLC:
Säule: Chiralpak AD-H/39, 5 µm, 250 x 4.6 mm
Laufmittel: Heptan : Ethanol 6 : 1 (30 °C, Fluss 1 ml / min)
Retentionszeiten: 8.383 Minuten (Enantiomer 1) und 12.467 Minuten (Enantiomer 2).

Die Verbindungen 31-34 konnten mit dieser Herstellungsvorschrift hergestellt werden:

### (S)-3-(4a,8a-Dimethyl-1,1-dioxo-4a,5,6,7,8,8a-hexahydro-1H-1lambda6-benzo[1,4,3]oxathiazin-3-ylamino)-3-phenyl-propan-1-ol

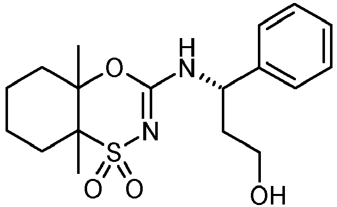

Zu einer Lösung aus enantiomerenreinen 4a,8a-Dimethyl-3-(4-nitro-phenoxy)-4a,5,6,7,8,8a-hexahydro-benzo[1,4,3]oxathiazin 1,1-dioxid Enantiomer 1 (50 mg) in Dichlormethan (2 ml) wurden S-3-Amino-3-phenylpropan-1-ol Hydrochlorid (30 mg) und Diisopropyl-ethylamin (50 mg) gegeben. Das Lösungsmittel wurde im Vakuum entfernt und der Rückstand 16 Stunden stehen gelassen. Anschließend wurde mit präparativer HPLC gereinigt. Man erhielt so das Produkt (32 mg) mit dem Molekulargewicht 366.4 g / mol (C₁₈H₂₆N₂O₄S), MS (ESI): m / e = 367 (M+H+).

### (S)-3-(4a,8a-Dimethyl-1,1-dioxo-4a,5,6,7,8,8a-hexahydro-1H-1lambda6-benzo[1,4,3]oxathiazin-3-ylamino)-3-pyridin-3-yl-propan-1-ol

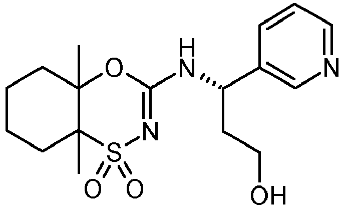

## Patentansprüche

1. Verbindungen der Formel I, 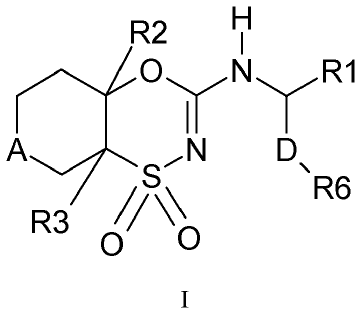 worin bedeuten
A CH₂, CF₂, O;
D (C₁-C₆)-Alkylen, (C₃-C₈)-Cycloalkylen, (C₁-C₆)-Alkylen-(C₃-C₈)-Cycloalkylen;
R1 -(CH₂)ₙ-Aryl,
wobei der Arylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
-(CH₂)ₙ-Heteroaryl,
wobei der Heteroarylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
n 0, 1, 2;
R2, R3 unabhängig voneinander H, (C₁-C₆)-Alkyl;
R6 OH, O-(CO)-NH₂, SO₂NH₂;
sowie deren pharmazeutisch verträgliche Salze.

2. Verbindungen der Formel I, gemäß Anspruch 1, **dadurch gekennzeichnet, dass** darin bedeuten
A CH₂;
D (C₁-C₆)-Alkylen, (C₃-C₈)-Cycloalkylen,
R1 -(CH₂)ₙ-Phenyl, -(CH₂)ₙ-Pyridinyl,
wobei der Phenylrest oder Pyridinylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH2, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
n 0, 1, 2;
R2, R3 unabhängig voneinander H, (C₁-C₆)-Alkyl
R6 OH, O-(CO)-NH₂, SO₂NH₂;
sowie deren pharmazeutisch verträgliche Salze.

3. Verbindungen der Formel I, gemäß Anspruch 1 oder 2, **dadurch gekennzeichnet, dass** darin bedeuten
A CH₂;
D (C₁-C₆)-Alkylen;
R1 Phenyl, Pyridin
wobei der Phenylrest oder Pyridinylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2, R3 unabhängig voneinander H, (C₁-C₆)-Alkyl
R6 OH;
sowie deren pharmazeutisch verträgliche Salze.

4. Verbindungen der Formel I, gemäß einem oder mehreren der Ansprüche 1 bis 3, **dadurch gekennzeichnet, dass** darin bedeuten
A CH₂;
D (C₁-C₂)-Alkylen;
R1 Phenyl,
wobei der Phenylrest ein bis 3-fach substituiert sein kann mit F, Cl, Br, I, OH, CF₃, CHF₂, CH₂F, NO₂, CN, OCF₃, OCHF₂, O-(C₁-C₆)-Alkyl, (C₁-C₆)-Alkyl, NH₂, NH(C₁-C₆)-Alkyl, N((C₁-C₆)-Alkyl)₂, SO₂-CH₃, SO₂-NH₂, SO₂-NH(C₁-C₆)-Alkyl, SO₂-N((C₁-C₆)-Alkyl)₂, COOH, COO-(C₁-C₆)-Alkyl, CONH₂, CONH(C₁-C₆)-Alkyl, CON((C₁-C₆)-Alkyl)₂, SF₅;
R2, R3 unabhängig voneinander H, (C₁-C₆)-Alkyl
R6 OH;
sowie deren pharmazeutisch verträgliche Salze.

5. Verbindungen der Formel I, gemäß einem oder mehreren der Ansprüche 1 bis 4, zur Anwendung als Arzneimittel.

6. Arzneimittel enthaltend Verbindungen der Formel I, gemäß einem oder mehreren der Ansprüche 1 bis 4.

7. Arzneimittel enthaltend Verbindungen der Formel I, gemäß einem oder mehreren der Ansprüche 1 bis 4 und mindestens einen weiteren Wirkstoff.

8. Arzneimittel, gemäß Anspruch 7, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CBI-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten, Lipase/Amylase-Inhibitoren, PPAR-Modulatoren, RXR-Modulatoren oder TR-β-Agonisten oder Amphetamine enthält.

9. Verfahren zur Herstellung eines Arzneimittels enthaltend die Verbindungen der Formel I, gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

10. Verwendung der Verbindungen der Formel I, gemäß einem oder mehreren der Ansprüche 1 bis 4 zur Herstellung eines Medikaments zur Behandlung von Hyperglykämie.

11. Verwendung der Verbindungen der Formel I, gemäß einem oder mehreren der Ansprüche 1 bis 4 zur Herstellung eines Medikaments zur Behandlung des Diabetes.

12. Verwendung der Verbindungen der Formel I, gemäß einem oder mehreren der Ansprüche 1 bis 4 zur Herstellung eines Medikaments zur Behandlung von Insulin Resistenz.

13. Set (Kit) bestehend aus getrennten Packungen von
a) einer wirksamen Menge einer Verbindung der Formel I, gemäß einem oder mehreren der Ansprüche 1 bis 4 und
b) einer wirksamen Menge eines weiteren Arzneimittelswirkstoffs.

## Claims

1. A compound of the formula I 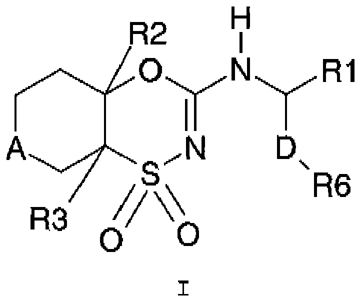 in which
A is CH₂, CF₂, O;
D is (C₁-C₆)-alkylene, (C₃-C₈)-cycloalkylene, (C₁-C₆)-alkylene-(C₃-C₈)-cycloalkylene;
R1 is - (CH₂)ₙ-aryl,
where the aryl radical may be mono- to trisubstituted by F, Cl, Br, I, OH, CF₃, CHF₂, CH₂F, NO₂, CN, OCF₃, OCHF₂, O-(C₁-C₆)-alkyl, (C₂-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
-(CH₂)ₙ-heteroaryl,
where the heteroaryl radical may be mono- to trisubstituted by F, Cl, Br, I, OH, CF₃, CHF₂, CH₂F, NO₂, CN, OCF₃, OCHF₂, O- (C₁-C₆)-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
n is 0, 1, 2;
R2, R3 are each independently H, (C₁-C₆)-alkyl;
R6 is OH, O-(CO)-NH₂, SO₂NH₂;
and pharmaceutically acceptable salts thereof.

2. A compound of the formula I as claimed in claim 1, **characterized in that**
A is CH₂;
D is (C₁-C₆) -alkylene, (C₃-C₈)-cycloalkylene;
R1 is - (CH₂)ₙ-phenyl, (CH₂)ₙ-pyridinyl,
where the phenyl radical or pyridinyl radical may be mono- to trisubstituted by F, Cl, Br, I, OH, CF₃, CRF², CH₂F, NO₂, CN, OCF₃, OCHF₂, O- (C₁-C₆) -alkyl, (C₁-C₆)-alkyl, NH₂, NH(C₁-C₆)-alkyl, N((C₁-C₆)-alkyl)₂, SO₂-CH₃, SO₂NH₂, SO₂-NH (C₁-C₆)-alkyl, SO₂-N((C₁-C₆)-alkyl)₂, COOH, COO-(C₁-C₆)-alkyl, CONH₂, CONH(C₁-C₆)-alkyl, CON((C₁-C₆)-alkyl)₂, SF₅;
n is 0, 1, 2;
R2, R3 are each independently H, (C₁-C₆)-alkyl;
R6 is OH, O-(CO)-NH₂, SO₂NH₂;
and pharmaceutically acceptable salts thereof.

3. A compound of the formula I as claimed in claim 1 or 2, **characterized in that**
A is CH₂;
D is (C₁-C₆)-alkylene;
R1 is phenyl, pyridine
where the phenyl radical or pyridinyl radical may be mono- to trisubstituted by F, Cl, Br, I, OH, CF₃, CHF₂, CH₂F, NO₂, CN, OCF₃, OCHF₂, O- (C₁-C₆) -alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆) -alkyl, SO₂-N(C₁-C₆)-alkyl)₂, COOH, COO-(C₁-C₆)-alkyl, CONH₂, CONH(C₁-C₆)-alkyl, CON((C₁-C₆)-alkyl)₂, SF₅;
R2, R3 are each independently H, (C₁-C₆)-alkyl;
R6 is OH;
and pharmaceutically acceptable salts thereof.

4. A compound of the formula I as claimed in one or more of claims 1 to 3, **characterized in that**
A is CH₂ ;
D is (C₁-C₂)-alkylene;
R1 is phenyl,
where the phenyl radical may be mono- to trisubstituted by F, Cl, Br, I, OH, CF₃, CHF₂, CH₂F, NO₂, CN, OCF₃, OCHF₂, O-(C₁-C₆-alkyl, (C₁-C₆)-alkyl, NH₂, NH(C₁-C₆)-alkyl, N((C₁-C₆)-alkyl)₂, SO₂-CH₃, SO₂-NH₂, SO₂-NH(C₁-C₆)-alkyl, SO₂-N((C₁-C₆)-alkyl)₂-COOH, COO-(C₁-C₆)-alkyl, CONH₂, CONH(C₁-C₆)-alkyl, CON((C₁-C₆)-alkyl)₂, SF₅;
R2, R3 are each independently H, (C₁-C₆)-alkyl;
R6 is OH;
and pharmaceutically acceptable salts thereof.

5. A compound of the formula I as claimed in one or more of claims 1 to 4 for use as a medicament.

6. A medicament comprising compounds of the formula I as claimed in one or more of claims 1 to 4.

7. A medicament comprising compounds of the formula I as claimed in one or more of claims 1 to 4 and at least one further active ingredient.

8. The medicament as claimed in claim 7, **characterized in that** it comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine:fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, GPR40 modulators, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists, lipase/amylase inhibitors, PPAR modulators, RXR modulators or TR β agonists or amphetamines.

9. A process for producing a medicament comprising the compounds of the formula I as claimed in one or more of claims 1 to 4, **characterized in that** the active ingredient is mixed with a pharmaceutically suitable carrier and this mixture is converted to a form suitable for administration.

10. The use of the compounds of the formula I as claimed in one or more of claims 1 to 4 for producing a medicament for treatment of hyperglycemia.

11. The use of the compounds of the formula I as claimed in one or more of claims 1 to 4 for producing a medicament for treatment of diabetes.

12. The use of the compounds of the formula I as claimed in one or more of claims 1 to 4 for producing a medicament for treatment of insulin resistance.

13. A set (kit) consisting of separate packages of
a) an effective amount of a compound of the formula I as claimed in one or more of claims 1 to 4 and
b) an effective amount of a further active medicament ingredient.

## Revendications

1. Composés de formule I 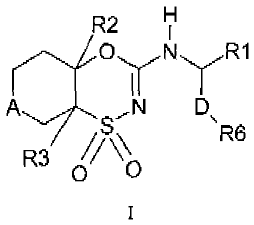 dans laquelle
A est CH₂, CF₂, O ;
D est un groupe alkylène en C₁-C₆, cycloalkylène en C₃-C₈, (alkylène en C₁-C₆)-(cycloalkylène en C₃-C₈);
R1 est un groupe -(CH₂)ₙ-aryle, dans lequel le radical aryle peut être substitué par un à 3 substituants F, Cl, Br, I, OH, CF₃, CHF₂, CH₂F, NO₂, CN, OCF₃, OCHF₂, O-(alkyle en C₂-C₆), alkyle en C₁-C₆, NH₂, NH(alkyle en C₁-C₆), N(alkyle en C₁-C₆)₂, SO₂CH₃, SO₂-NH₂, SO₂-NH(alkyle en C₁-C₆), SO₂-N(alkyle en C₁-C₆)₂, COOH, COO-(alkyle) en C₁-C₆), CONH₂, CONH(alkyle en C₁-C₆), CON(alkyle en C₁-C₆)₂, SF₅ ;
un groupe -(CH₂)ₙ-hétéroaryle, dans lequel le radical hétéroaryle peut être substitué par un ou 3 substituants F, Cl, Br, I, OH, CF₃, CHF₂, CH₂F, NO₂, CN, OCF₃, OCHF₂, O-(alkyle en C₁-C₆), alkyle en C₁-C₆, NH₂, NH(alkyle en C₁-C₆), N(alkyle en C₁-C₆)₂, SO₂CH₃, SO₂-NH₂, SO₂-NH(alkyle en C₁-C₆), SO₂-N(alkyle en C₁-C₆)₂, COOH, COO-(alkyle en C₁-C₆), CONH₂, CONH(alkyle en C₁-C₆), CON(alkyle en C₁-C₆)₂, SF₅ ;
n vaut 0, 1, 2 ;
R2, R3 représentent chacun indépendamment de l'autre H, un groupe alkyle en C₁-C₆ ;
R6 est OH, O-(CO)-NH₂, SO₂NH₂ ;
ainsi que leurs sels pharmaceutiquement acceptables.

2. Composés de formule I selon la revendication 1, **caractérisés en ce que**, dans cette formule,
A est CH₂ ;
D est un groupe alkylène en C₁-C₆, cycloalkylène en C₃-C₉ ;
R1 est un groupe - (CH₂)ₙ-phényle, - (CH₂)ₙ-pyridinyle, le radical phényle ou le radical pyridinyle pouvant être substitué par un à 3 substituants F, Cl, Br, I, OH, CF₃, CHF₂, CH₂F, NO₂, CN, OCF₃, OCHF₂, O-(alkyle en C₁-C₆), alkyle en C₁-C₆, NH₂, NH(alkyle en C₁-C₆), N(alkyle en C₁-C₆)₂, SO₂CH₃, SO₂-NH₂, SO₂-NH(alkyle en C₁-C₆), SO₂-N(alkyle en C₁-C₆)₂, COOH, COO(alkyle en C₁-C₆), CONH₂, CONH(alkyle en C₁-C₆), CON(alkyle en C₁-C₆)₂, SF₅ ;
n vaut 0, 1, 2 ;
R2, R3 représentent chacun indépendamment de l'autre H, un groupe alkyle en C₁-C₆ ;
R6 est OH, O-(CO)-NH₂, SO₂NH₂ ;
ainsi que leurs sels pharmaceutiquement acceptables.

3. Composés de formule I selon la revendication 1 ou 2. **caractérisés en ce que**, dans cette formule
A est CH₂ ;
D est un groupe alkylène en C₁-C₆ ;
R1 est un groupe phényle, pyridinyle, le radical phényle ou le radical pyridinyle pouvant être substitué par un à 3 substituants F, Cl, Br, I, OH, CF₃, CHF₂, CH₂F, NO₂, CN, OCF₃, OCHF₂, O-alkyle en C₁-C₆), alkyle en C₁-C₆, NH₂, NH(alkyle en C₁-C₆), N(alkyle en C₁-C₆)₂, SO₂CH₃, SO₂-NH₂, SO₂-NH(alkyle en C₁-C₆), SO₂-N(alkyle en C₁-C₆)₂, COOH, COO-(alkyle en C₁-C₆), CONH₂, CONH(alkyle en C₁-C₆), CON(alkyle en C₁-C₆)₂, SF₅ ;
R2, R3 représentent chacun indépendamment de l'autre H, un groupe alkyle en C₁-C₆ ;
R6 est OH ;
ainsi que leurs sels pharmaceutiquement acceptables.

4. Composés de formule 1 selon l'une ou plusieurs des revendications 1 à 3, **caractérisés en ce que**, dans cette formule,
A est CH₂ ;
D est un groupe alkylène en C₁-C₂ ;
R1 est un groupe phényle, le radical phényle pouvant être substitué par un à 3 substituants F, Cl, Br, I, OH, CF₃, CHF₂, CH₂F, NO₂, CN, OCF₃, OCHF₂, O-(alkyle en C₁-C₆), alkyle en C₁-C₆, NH₂, NH(alkyle en C₁-C₆),
N(alkyle en C₁-C₆)₂, SO₂CH₃, SO₂-NH₂, SO₂-NH(alkyle en C₁-C₆), SO₂-N(alkyle en C₁-C₆)₂, COOH, COO-(alkyle en C₁-C₆), CONH₂, CONH (alkyle en C₁-C₆), CON(alkyle en C₁-C₆)₂, SF₅ ; R2, R3 représentent chacun indépendamment de l'autre H, un groupe alkyle en C₁-C₆ ;
R6 est OH ;
ainsi que leurs sels pharmaceutiquement acceptables.

5. Composés de formule I selon l'une ou plusieurs des revendications 1 à 4 pour une utilisation en tant que médicament.

6. Médicament contenant des composés de formule I selon l'une ou plusieurs des revendications 1 à 4.

7. Médicament contenant des composés de formule I selon l'une ou plusieurs des revendications 1 à 4 et au moins un autre principe actif.

8. Médicament selon la revendication 7, **caractérisé en ce qu'**il contient en tant qu'autre principe actif un ou plusieurs antidiabétiques, principes actifs hypoglycémiques, inhibiteurs de la HMGCoA-réductase, inhibiteurs de la résorption du cholestérol, agonistes du PPAR gamma, agonistes du PPAR alpha, agonistes du PPAR alpha/gamma, agonistes du PPAR delta, fibrates, inhibiteurs de la MTP, inhibiteurs de la résorption des acides biliaires, inhibiteurs de la CETP, agents polymères adsorbant les acides biliaires, inducteurs des récepteurs des LDL, inhibiteurs de l'ACAT, antioxydants, inhibiteurs de la lipoprotéine-lipase, inhibiteurs de l'ATP-citrate-lyase, inhibiteurs de la squalène synthétase, antagonistes de la lipoprotéine(a), agonistes du récepteur HM74A, inhibiteurs de la lipase, insulines, sulfonylurées, biguanide, méglitinide, thiazolidinedione, inhibiteurs de l'α-glucosidase, principes actifs agissant sur le canal potassique ATP-dépendant des cellules bêta, inhibiteurs de la glycogène phosphorylase, antagonistes du récepteur du glucagon, activateurs de la glucokinase, inhibiteurs de la gluconéogenèse, inhibiteurs de la fructose-1,6-biphosphatase, modulateurs du transporteur de glucose de type 4, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur de glucose sodium-dépendant 1 ou 2, modulateurs de la GPR40, inhibiteurs de la lipase sensible aux hormones, inhibiteurs de l'acétyl-CoA-carboxylase, inhibiteurs de la phosphoénol-pyruvatecarboxykinase, inhibiteurs de la glycogène synthase kinase-3 bêta, inhibiteurs de la protéine kinase C bêta, antagonistes du récepteur de l'endothéline-A, inhibiteurs de la I kappaB kinase, modulateurs du récepteur des glucocorticoïdes, agonistes du CART, agonistes du NPY, agonistes du MC4, agonistes de l'oréxine, agonistes de H3, agonistes du TNF, agonistes du CRF, antagonistes du CRF BP, agonistes de l'urocortine, agonistes de β3, antagonistes du récepteur CB1, agonistes de la MSH (hormone de stimulation des mélanocytes), agonistes du CCK, inhibiteurs de la recapture de la sérotonine, composés de sérotonine et noradrénergiques mixtes, agonistes de 5HT, agonistes de la bombésine, antagonistes de la galanine, hormones de croissance, composés libérant l'hormone de croissance, agonistes de TRH, modulateurs de la protéine de découplage 2 ou 3, agonistes de la leptine, agonistes de la DA, inhibiteurs de la lipase-amylase, modulateurs de PPAR, modulateurs des RXR ou agonistes des TR-β, ou amphétamines.

9. Procédé de fabrication d'un médicament contenant les composés de formule I selon l'une ou plusieurs des revendications 1 à 4, **caractérisé en ce que** le principe actif est mélangé à un support pharmaceutiquement approprié, et que ce mélange est mis sous une forme convenant à l'administration.

10. Utilisation des composés de formule I selon l'une ou plusieurs des revendications 1 à 4 pour fabriquer un médicament destiné au traitement de l'hyperglycémie.

11. Utilisation des composés de formule I selon l'une ou plusieurs des revendications 1 à 4, pour fabriquer un médicament destiné au traitement du diabète.

12. Utilisation des composés de formule I selon l'une ou plusieurs des revendications 1 à 4, pour fabriquer un médicament destiné au traitement de l'insulino-résistance.

13. Trousse (kit) consistant en des conditionnements distincts
a) d'une quantité efficace d'un composé de formule I selon l'une ou plusieurs des revendications 1 à 4, et
b) d'une quantité efficace d'un autre principe actif médicamenteux.
